(19) **Europäisches Patentamt**
**European Patent Office**
**Office européen des brevets**

(11) **EP 3 415 651 A1**

(12) **EUROPEAN PATENT APPLICATION**

(43) Date of publication:
**19.12.2018 Bulletin 2018/51**

(51) Int Cl.:
***C22C 19/05*** (2006.01)

(21) Application number: **17175929.3**

(22) Date of filing: **14.06.2017**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(71) Applicants:
• **Heraeus Deutschland GmbH & Co. KG
63450 Hanau (DE)**
• **Saes Smart Materials, Inc.
New Hartford, NY 13413 (US)**
• **Heraeus Materials Singapore Pte. Ltd.
569881 Singapore (SG)**
• **Heraeus Medical Components, LLC
St. Paul, MN 55127 (US)**

(72) Inventors:
• **RICHTER, René
64832 Babenhausen (DE)**

• **GEBERT, Dr. Jörg-Martin
76227 Karlsruhe (DE)**
• **SPECHT, Heiko
63456 Hanau (DE)**
• **SCZERZENIE, Francis E.
Lake Pleasant, NY New York 12108 (US)**
• **MANJERI, Radhakrishnan M.
New Hartford, NY New York 13413-2250 (US)**
• **YIN, Weimin
Ridgefield, CT Connecticut 06877 (US)**
• **GOLAGANI VENKATA KANAKA, Sai Srikanth
730586 Singapore (SG)**
• **LARK, Larry
Saint Paul, MN Minnesota 55116 (US)**

(74) Representative: **Maiwald Patent- und
Rechtsanwaltsgesellschaft mbH
Elisenhof
Elisenstraße 3
80335 München (DE)**

(54) **A METHOD FOR MANUFACTURING A PASSIVATED PRODUCT**

(57)    The invention generally relates to a method for manufacturing a passivated product, a passivated product, and a medical device comprising such passivated product. The passivated product comprises a raw product and a passivation coating. The raw product is made at least partially of an alloy comprising Cr, Ni, Mo and Co, preferably with tightly controlled levels of impurities. The method for manufacturing the passivated product comprises a providing of a raw product and a passivating of a surface of the raw product at least partially to provide a passivated product with a passivation coating. The raw product is made at least partially of an alloy comprising the following alloy components:

a) Cr in the range from about 10 to about 30 wt. %;
b) Ni in the range from about 20 to about 50 wt. %;
c) Mo in the range from about 2 to about 20 wt. %;
d) Co in the range from about 10 to about 50 wt. %.

The A1 content of the Cr, Ni, Mo and Co alloy is less than about 0.01 wt. % and each wt. % is based on the total weight of the alloy.

Fig. 14

**Description**

Field of the invention

The invention generally relates to a method for manufacturing a passivated product, a passivated product, and a medical device comprising such passivated product. The passivated product comprises a raw product and a passivation coating. The raw product is made at least partially of an alloy comprising Cr, Ni, Mo and Co, preferably with tightly controlled levels of impurities.

Background of the invention

Much investigation in recent years has been directed to a search for new high performance alloys, particularly for medical applications where a very high value is placed on reliability and materials are required which exhibit a low failure rate even over a long time period.

Cardiac Pacemakers, Implantable Cardioverter Defibrillation Devices and Cardiac Resynchronisation Devices are applications where reliability is particularly important, especially in terms of resistance to physical fatigue and to chemical corrosion. Invasive surgery is required to implant a pacemaker into the body or remove or replace parts, and it is highly desirable for the individual components of the pacemaker to have a long working life in order to reduce the requirement for surgical intervention. Furthermore, it is desirable for the working life to have a low variance. In a heart pacemaker, one component, which is exposed to a particularly high amount of stress during normal operation is the so called lead which connects the implantable pulse generator to the heart tissue. A flexible lead is required in order to connect the implantable pulse generator to the heart tissue without imposing undue physical stress on the heart and the lead flexes during normal operation, typically repetitively with a frequency on the order of that of a human heart beat. A high resistance to fatigue is therefore required in the lead in order to withstand frequent physical stress over a long period of time. A high resistance of the lead to corrosion is important not only in terms of the lifetime of the component, but also in terms of reducing toxicity to the body.

WO 2005026399 A1 discusses an approach to improving the properties of an alloy by reducing the content of titanium nitride and mixed metal carbonitride.

US 2005/0051243 A1 focuses on alloys with a reduced content of nitrogen.

A manufacturing of wires may comprise a drawing of a rod or coil from larger diameters to subsequently smaller diameters. The manufacturing may comprise single die drawing on a drawing bench or on a bull block. With subsequently smaller diameters, the wire may be drawn by multi-die-drawing.

Summary of the invention

There may be a need to provide an improved manufacturing method, which in particular may increase a product's life span.

The problem of the present invention is solved by the subject-matters of the independent claims, wherein further embodiments are incorporated in the dependent claims. It should be noted that the aspects of the invention described in the following apply also to the method for manufacturing a passivated product, the passivated product, and the medical device comprising such passivated product.

According to the present invention, a method for manufacturing a passivated product is presented. The method for manufacturing the passivated product comprises the following steps, not necessarily in this order:

- providing a raw product, and
- passivating a surface of the raw product at least partially to provide a passivated product with a passivation coating.

The raw product is made at least partially of an alloy comprising the following alloy components:

a) Cr in the range from about 10 to about 30 wt. %;
b) Ni in the range from about 20 to about 50 wt. %;
c) Mo in the range from about 2 to about 20 wt. %;
d) Co in the range from about 10 to about 50 wt. %.

The Al content of the Cr, Ni, Mo and Co alloy is less than about 0.01 wt. % and each wt. % is based on the total weight of the alloy.

The raw product may be a semi-finished material, for instance a rod or coil. The present invention may relate to a passivation of a surface of the raw product. The passivated product may be the raw product with the passivation coating. Passivation may mean that the raw product spontaneously forms a chemically inactive surface when exposed to air, moisture or other oxygen-containing environments. Passivation may also mean a chemical treatment of the raw product with a mild oxidant, such as a nitric acid solution, for enhancing or augmenting the spontaneous formation of a protective passive film without significantly affecting the raw product itself. Such chemical treatment is generally not necessary for the formation of the passive film. Passivation can also be understood as an application of a thin and dense coating or layer around the bare metal of the raw product. This can be for instance an oxide layer of one of the alloying elements of the raw product or an oxide layer of a noble metal, which are both fully dense and very thin. "Thin" means here in the range of a few angstrom as for example for noble metals (e.g. Platinum group metals) or more in the range of several microns for less noble metals (e.g. Aluminum alloys).

The Cr, Ni, Mo and Co alloy may be an alloy, which has improved resistance to physical fatigue, a high corrosion resistance and/or the ability to be drawn into a thin wire. In an example, the Cr, Ni, Mo and Co components are major constituents of the Cr, Ni, Mo and Co alloy with at least about 95 wt. % of the alloy being Cr, Ni, Mo and Co. Details in view of the alloy are provided further below.

The use of the passivation coating on the Cr, Ni, Mo and Co alloy prior to another manufacturing step may reduce friction between the passivated product and a manufacturing tool. As a result, shear stresses close to the product surface are reduced, which may otherwise damage a surface volume of the product and introduce surface defects. Due to less surface defects, the surface quality is maintained longer and the product's fatigue life is further increased, as the origin of failure is mainly due to surface defects. Fatigue life is especially important for medical applications where a product may be exposed up to several hundred million cycles of mechanical bending. Further, the passivation coating may further improve a corrosion resistance of the product. Furthermore, manufacturing costs and therefore also product costs can be reduced as a life time of the manufacturing tool is increased due to less friction between the product and the manufacturing tool.

In an example, the passivation coating is configured to provide a full coverage of the surface of the raw product. The passivation coating may then cover and protect the entire passivated product during other manufacturing steps and/or during operation. The passivation coating may reduce or even inhibit a direct contact between the raw product within the passivation coating and e.g. a manufacturing tool.

In an example, the product is a wire. The raw product may be a raw wire and the passivated product may be a passivated wire. The wire may be a single strand or rod of metal or may comprise a bundle of such strands or rods. The wire may be configured to bear mechanical loads or electricity and/or telecommunications signals. The wire may be flexible and may be circular in cross-section or square, hexagonal, flattened, rectangular or the like.

In an example, the method further comprises a wire drawing of the passivated wire. The passivation coating may be configured to provide a full coverage of the surface of the raw wire, which is maintained during wire drawing and prevents a direct contact between the raw wire and a drawing tool. The passivation coating may then be drawn and deformed with the bulk material and maintains its complete coverage of the metal surface. This way, the alloy of the raw wire is not in direct contact to the drawing die and friction between wire and die is reduced as the friction coefficient of oxide layer to die material is lower compared to bare alloy to die material. As the friction is reduced, also shear stresses and consequently surface defects close to the wire surface are reduced. Due to less surface defects, the wire's fatigue life is increased, as the origin of failure are mainly surface defects.

In an example, the passivation coating is provided directly on the metallic surface of the raw product. This means there is no other material or layer between the metallic surface of the raw product and the passivation coating.

In an example, the passivation coating is inert. The passivation coating may be inert in conditions during further manufacturing steps and/or during operation. The passivation coating may be inert in conditions during wire drawing. The passivation coating may be inert when exposed to air and in particular when exposed to oxygen. The passivation coating may also be inert when exposed to room temperature, body temperature or elevated temperatures during further manufacturing steps as e.g. annealing.

In an example, the passivation coating has a thickness in the range of 10 nm to 50 $\mu$m, and preferably in the range of 50 nm to 10 $\mu$m. The thickness of the passivation coating is then high enough to effectively protect the alloy surface but thin enough to deform with the bulk alloy during wire drawing.

In an example, the passivation coating is an oxide layer of an oxide of one of the group of Cr, Ni, Mo and Co. In an example, the passivation coating is a chromium oxide layer. In another example, the passivation coating is an oxide layer of an oxide of a noble metal. In an example, the passivation coating is a platinum oxide layer. All above mentioned oxide layers are dense, thin and inert to protect the alloy from any strain introduced by e.g. a mechanical manufacturing tool.

In an example, the raw product or raw wire is annealed before passivating. In other words, a stock material used for the manufacturing method according to the invention can be a rolled and coiled material in fully annealed condition.

In an example, the passivating occurs automatically or spontaneously, which means without any man made efforts. In another example, the passivating is enhanced or assisted by means of a chemical agent, e.g. an oxidizing agent or an acid. In an example, the passivating is done by means of a nitric acid solution. Passivation may be done by e.g. 20 to 25 volume percent of nitric acid and 2.5 $\pm$ 0.5 weight percent of sodium dichromate for about 20 minutes at a temperature in the range from 50 to 55°C, 20 to 45 volume percent of nitric acid for about 30 minutes at a temperature in the range from 20 to 30°C, 20 to 25 volume percent nitric acid for about 20 minutes at a temperature in the range from 50 to 60°C, 45 to 55 volume percent of nitric acid for about 30 minutes at a temperature in the range from 50 to 55°C, 4 to 10 weight percent of citric acid for about 4 minutes at a temperature in the range from 60 to 70°C, 4 to 10 weight percent of citric acid for about 10 minutes at a temperature in the range from 50 to 60°C, 4 to 10 weight percent of citric acid for about 20 minutes at a temperature in the range from 20 to 50°C or the like. The passivation may be performed as described in ASTM F86 - 13 (Standard Practice for Surface Preparation and Marking of Metallic Surgical Implants, 2013), ASTM A380 / ASTM A380M - 13 (Standard Practice for Cleaning, Descaling, and Passivation of Stainless Steel Parts, Equipment, and Systems, 2013), ASTM A967 / ASTM A967M - 13 (Chemical Passivation Treatments for Stainless Steel Parts, 2013) or the like.

In an example, the method for manufacturing a passivated product further comprises a cleaning of the raw product before passivating. In an example, the cleaning comprises one of a group of organic solvent degassing, hot alkaline cleaning and acidic cleaning. The effect may be that any residual lubricants are removed from the raw product before passivating to improve the quality of the passivating coating. The cleaning of contaminants from the raw product surface may further facilitate a spontaneous or chemically assisted passivation by allowing a uniform access of oxygen to the surface.

In case of an acidic cleaning, the product may need to be neutralized prior to passivation. In an example, the method for manufacturing a passivated product therefore further comprises a neutralizing of the raw product after cleaning and before passivating.

The chemical reactions of the passivating media on the surface of the passivated product can be stopped by rinsing of the passivated product. In an example, the method for manufacturing a passivated product therefore comprises a rinsing of the passivated product after passivating. In an example, the method for manufacturing a passivated product further comprises an electrical monitoring of the rinsing agent e.g. DI water after rinsing. The electrical monitoring may ensure that the rinse is completed properly, which may be shown by monitoring an electrical conductivity of the rinse agent after passing the product.

If an (intermediate) heat treatment shall be conducted, the passivation coating may be removed mechanical and/or chemical before the heat treatment and may be re-applied after the heat treatment. In an example, the method for manufacturing a passivated product therefore further comprises a removing of the passivation coating, a heat-treating of the product and a repassivating of the heat-treated product.

In another example, the method for manufacturing a passivated product further comprises an additional, e.g. second passivating of the drawn wire and/or an additional, e.g. second wire drawing of the again passivated wire. In other words, the passivation coating on the passivated product may be renewed after the (first) wire drawing. Specifically, this could done in-line (from spool to spool) in a sequences of baths with or without ultrasonic followed by a rinse with e.g. DI water, followed by a cleaning with e.g. alcohol.

According to the present invention, also a passivated product comprising a raw product and a passivation coating is presented. The passivation coating is provided at least partially on a surface of the raw product and the raw product is made at least partially of an alloy comprising the following alloy components:

    a) Cr in the range from about 10 to about 30 wt. %;
    b) Ni in the range from about 20 to about 50 wt. %;
    c) Mo in the range from about 2 to about 20 wt. %;
    d) Co in the range from about 10 to about 50 wt. %.

The Al content of the alloy is less than about 0.01 wt. % and each wt. % is based on the total weight of the alloy. The passivated product may be a semi-finished or finished article used for medical applications. Such articles may include medical wires, solid or clad wires, round wires, profiles, flat wires, or other articles made thereof.

According to the present invention, also a passivated product manufactured by the method as described above is presented.

The raw product may be a raw composite wire, which may be understood as a wire comprising at least two different materials. One of these materials may be the Cr, Ni, Mo and Co alloy described above. The other material may be different to this alloy. The Cr, Ni, Mo and Co alloy may form an inner volume of the composite wire and the other material may form an outer volume of the composite wire when seen in a cross section. The Cr, Ni, Mo and Co alloy may also form the outer volume of the composite wire and the other material may form the inner volume of the composite wire when seen in a cross section.

The other material can be a metallic material as e.g. a metal or another alloy. It may comprise at least one of a group of Silver, Platinum, Tantalum, Gold, Copper and alloys thereof. The other material may comprise at least one of a group of: Platinum, a Platinum based alloy, a Platinum-Iridium alloy, a Platinum-Tungsten alloy, Gold, a Gold alloy, Tantalum, Titanium, a Titanium-Molybdenum alloy, and a Titanium Aluminum Vanadium alloy. It may comprise a Platinum-Iridium alloy with about 70-90 wt. % Platinum and 10-30 wt. % Iridium.

According to the present invention, a medical device comprising a passivated product as described above as a lead is presented. The medical device may be a pacemaker, an implantable cardioverter defibrillator, a cardiac resyncronisation device, a neuromodulation device, a cochlea implant or any other implantable stimulation device comprising a composite wire as described above as a lead.

The Cr, Ni, Mo and Co alloy may be an alloy, which has improved resistance to physical fatigue, a high corrosion resistance, and/or which can be drawn into a thin wire, preferably less than about 50 μm. The wire according to the invention may be a wire having comparable tensile properties to known wires, but for which the proportion of outlying failures in fatigue resistance is reduced.

A contribution to achieving at least one of the above described objects is made by the following embodiments of the Cr, Ni, Mo and Co alloy (in the following "alloy").

|1| An alloy comprising the following alloy components:

a) Cr in the range from about 10 to about 30 wt. %, preferably in the range from about 15 to about 25 wt. %, more preferably in the range from about 19 to about 21 wt. %;
b) Ni in the range from about 20 to about 50 wt. %, preferably in the range from about 30 to about 45 wt. %, more preferably in the range from about 33 to about 37 wt. %;
c) Mo in the range from about 2 to about 20 wt. %, preferably in the range from about 5 to about 15 wt. %, more preferably in the range from about 9 to about 10.5 wt. %;
d) Co in the range from about 10 to about 50 wt. %, preferably in the range from about 20 to about 40 wt. %, more preferably in the range from about 33 to about 37 wt. %;

wherein the Al content of the alloy is less than about 0.01 wt. %, preferably less than about 0.005 wt. %, more preferably less than about 0.001 wt. %;
wherein each wt. % is based on the total weight of the alloy.

|2| The alloy according to embodiment |1|, wherein the content of Mg is less than about 0.005 wt. %, preferably less than about 0.0001 wt. %, more preferably less than about 0.00001 wt. %, based on the total weight of the alloy.

|3| The alloy according to embodiment |1| or |2|, wherein the content of Ca is less than about 0.005 wt. %, preferably less than about 0.0001 wt. % more preferably less than about 0.00001 wt. %, based on the total weight of the alloy.

|4| The alloy according to any of the preceding embodiments, wherein the content of Ce is less than about 0.005 wt. %, preferably less than about 0.0001 wt. % more preferably less than about 0.00001 wt. %, based on the total weight of the alloy.

|5| The alloy according to any of the preceding embodiments, wherein the content of Ti is less than about 0.1 wt. %, preferably less than about 0.01 wt. % more preferably less than about 0.001 wt. %, further more preferably less than about 0.0005 wt. %, based on the total weight of the alloy.

|6| The alloy according to any of the preceding embodiments, wherein the content of Fe is in the range from about 0.0001 to about 1 wt. %, preferably in the range from about 0.0005 to about 0.1 wt. %, more preferably in the range from about 0.001 to about 0.05 wt. %, based on the total weight of the alloy.

|7| The alloy according to any of the preceding embodiments, wherein at least one of the following is satisfied:

a) The content of C in the alloy is less than about 0.1 wt. %, preferably less than about 0.08 wt. % more preferably less than about 0.05 wt. %
b) The content of B in the alloy is less than about 0.01 wt. %, preferably less than about 0.001 wt%, more preferably less than about 0.0002 wt. %;
c) The content of P in the alloy is less than about 0.01 wt. %, preferably less than about 0.005 wt. %, more preferably less than about 0.001 wt. %, further more preferably less than about 0.0005 wt. %;
d) The content of S in the alloy is less than about 0.005 wt. %, preferably less than about 0.003 wt. %, more preferably less than about 0.002 wt. %, further more preferably less than about 0.0008 wt. %;

each wt. % being based on the total weight of the alloy. In preferred aspects of this embodiment, the combination of the above criteria which are satisfied is selected from the group consisting of: a), b), c), d), a)+b), a)+c), a)+d), b)+c), b)+d), c)+d), a)+b)+c), a)+b)+d), a)+c)+d), b)+c)+d) and a)+b)+c)+d).

|8| The alloy according to any of the preceding embodiments, wherein at least one of the following is satisfied:

a) The content of Mn in the alloy is less than about 0.05 wt. %, preferably less than about 0.005 wt. %, more preferably less than about 0.001 wt. %;
b) The content of Si in the alloy is less than about 0.05 wt. %, preferably less than about 0.03 wt. %, more preferably less than about 0.02 wt. %;

each wt. % being based on the total weight of the alloy. In preferred aspects of this embodiment, the combination of the above criteria which are satisfied is selected from the group consisting of: a), b), a)+b).

|9| The alloy according to any of the preceding embodiments, wherein at least one of the following is satisfied:

a) The content of O in the alloy is in the range from about 0.0001 to about 0.05 wt. %, preferably in the range from about 0.0001 to about 0.03 wt. %, more preferably in the range from about 0.0001 to about 0.01 wt. %;
b) The content of N in the alloy is in the range from about 0.0001 to about 0.01 wt. %, preferably in the range from about 0.0001 to about 0.008 wt. %, more preferably in the range from about 0.0001 to about 0.005 wt. %;

each wt. % being based on the total weight of the alloy. In preferred aspects of this embodiment, the combination of the above criteria which are satisfied is selected from the group consisting of: a), b), a)+b).

|10| The alloy according to any of the preceding embodiments, wherein at least one of the following is satisfied:

a) The alloy contains less than about 0.01 wt. %, preferably less than about 0.005 wt. %, more preferably less than about 0.001 wt. %, O in the form of a magnesium oxide;
b) The alloy contains less than about 0.01 wt. %, preferably less than about 0.005 wt. %, more preferably less than about 0.001 wt. %, O in the form of an aluminium oxide;
c) The alloy contains less than about 0.01 wt. %, preferably less than about 0.005 wt. %, more preferably less than about 0.001 wt. %, O in the form of a cerium oxide.
d) The alloy contains less than about 0.01 wt. %, preferably less than about 0.005 wt. %, more preferably less than about 0.001 wt. %, O in the form of a calcium oxide.
e) The alloy contains less than about 0.01 wt. %, preferably less than about 0.005 wt. %, more preferably less than about 0.001 wt. %, O in the form of a chromium oxide.

In preferred aspects of this embodiment, the combination of the above criteria which are satisfied is selected from the group consisting of: a), b), c), d), a)+b), a)+c), a)+d), b)+c), b)+d), c)+d), a)+b)+c), a)+b)+d), a)+c)+d), b)+c)+d), a)+b)+c)+d), e), a)+e), b)+e), c)+e), d)+e), a)+b)+e), a)+c)+e), a)+d)+e), b)+c)+e), b)+d)+e), c)+d)+e), a)+b)+c)+e), a)+b)+d)+e), a)+c)+d)+e), b)+c)+d)+e) and a)+b)+c)+d)+e).

|11| A process for the preparation of an alloy comprising the following preparation steps:

a) Provision of a mixture comprising the following components:

i. Cr in the range from about 10 to about 30 wt. %, preferably in the range from about 15 to about 25 wt. %, more preferably in the range from about 19 to about 21 wt. %;
ii. Ni in the range from about 20 to about 50 wt. %, preferably in the range from about 25 to about 40 wt. %, more preferably in the range from about 33 to about 37 wt. %;
iii. Mo in the range from about 2 to about 20 wt. %, preferably in the range from about 5 to about 15 wt. %, more preferably in the range from about 9 to about 10.5 wt. %;
iv. Co in the range from about 10 to about 50 wt. %, preferably in the range from about 20 to about 40 wt. %, more preferably in the range from about 33 to about 37 wt. %.

wherein each wt. % is based on the total weight of the mixture prepared for melting;
b) Melting the mixture in a vacuum induction melting step in order to obtain a first melt, in one aspect of this embodiment, one or more further vacuum induction melting steps are carried out;

c) Solidifying the first melt in order to obtain a first solid;
d) Melting the first solid in a vacuum arc melting step in order to obtain a further melt;
e) Solidifying the further melt in order to obtain a further solid.

|12| The process according to embodiment |11|, wherein pressure in step b) is below about 0.1 bar, preferably below about 0.05 bar, more preferably below about 0.01 bar.

|13| The process according to embodiment |11| or |12|, wherein the leak rate in step b) is below about 0.1 bar / min, preferably below about 0.05 bar / min, more preferably below about 0.01 bar / min.

|14| The process according to any of the embodiments |11| to |13|, wherein the pressure in step d) is below about 0.05 bar, preferably below about 0.01 bar, more preferably below about 0.005 bar.

|15| The process according to any of the embodiments |11| to |14|, wherein the leak rate in step d) is below about 0.05 bar / min, preferably less than about 0.01 bar / min, more preferably less than about 0.005 bar / min.

|16| The process according to any of the embodiments |11| to |15|, further comprising a homogenisation step carried out at a temperature in the range from about 900 to about 1300 °C, preferably in the range from about 1000 to about 1250 °C, more preferably in the range from about 1100 to about 1225 °C.

|17|, The process according to any of the embodiments |11| to |16|, further comprising a cogging step carried out at a temperature in the range from about 900 to about 1300 °C, preferably in the range from about 1000 to about 1250 °C, more preferably in the range from about 1100 to about 1225 °C.

|18| The process according to any of the embodiments |11| to |17|, further comprising a finish roll step carried out at a temperature in the range from about 900 to about 1300 °C, preferably in the range from about 1000 to about 1250 °C, more preferably in the range from about 1100 to about 1225 °C.

|19| The process according to any of the embodiments |11| to |18|, further comprising a straightening step. In one aspect of this embodiment, the straightening is a hot straightening, preferably carried out at a temperature in the range from about 900 to about 1200 °C, preferably in the range from about 950 to about 1100 °C, more preferably in the range from about 1000 to about 1075 °C. In one aspect of this embodiment, the straightening is a cold straightening, preferably carried out at ambient temperature, preferably at a temperature in the range from about 10 to about 100 °C, more preferably in the range from about 15 to about 80 °C, most preferably in the range from about 20 to about 50 °C.

|20| An alloy obtainable by a process according to any of the embodiments |11| to |19|.

|21| An electrical wire comprising an alloy according to any of the embodiments |1| to |10| or |20|.

|22| A medical device comprising a wire according to embodiment |21|.

|23| A pacemaker, an implantable cardioverter defibrillator, a cardiac resyncronisation device, a neuromodulation device, a cochlea implant or any other implantable stimulation device comprising a wire according to embodiment |21|.

Alloy

The Cr, Ni, Mo and Co alloy comprise two or more elements, preferably as a solid mixture, preferably with an enthalpy of mixing of the constituent elements of less than about 10 KJ/mol, preferably less than about 5 KJ/mol, more preferably less than about 1 KJ/mol. The Cr, Ni, Mo and Co alloy comprise Cr, Ni, Mo and Co as major constituents, preferably with at least about 95 wt. %, more preferably at least about 99 wt. %, further more preferably at least about 99.9 wt. %, more preferably at least about 99.95 wt. % of the alloy being Cr, Ni, Mo and Co.

A composition of the Cr, Ni, Mo and Co alloy is preferred which improves favourable properties of the alloy, in particular resistance to fatigue and/or corrosion resistance, preferably both.

It is preferred for the properties of the alloy to be improved by limiting the content of impurities or limiting the content of a combination of different impurities, preferably according the embodiments of the invention.

It is preferred that there be a low, preferably zero concentration of inclusions in the alloy. This is preferably achieved by limiting the content of impurities. In one embodiment it is preferred that the alloy contain less than about 0.01 %, preferably less than about 0.005 %, more preferably less than about 0.001 % inclusions. The % of inclusions is preferably determined using the microscopic inspection method given in the test methods. Content of inclusions as % is there determined as the proportion of the cross sectional area of the sample surface made up of inclusions. In some instances, the alloy comprises a low, preferably a zero concentration of inorganic non-metallic solid inclusions, more preferably of inorganic oxide inclusions. Inorganic oxides in this context can refer to metal oxides, non-metal oxides and metalloid-oxides. In some cases the the alloy comprises a low, preferably a zero concentration of inclusions comprising one or more selected from the group consisting of: Si, Al, Ti, Zr and B; preferably selected form the group consisting of: Si Ti, and Al.

In one embodiment, one or more treating material(s) is/are contacted with the mixture of the process in order to remove oxygen from the mixture of the process, preferably by incorporation of the oxygen into a dross and removal of the dross. Preferred treating materials in this context comprise one or more selected from the list consisting of: Al, Mg, Ca and Ce; preferably in the form of an element and/or in the form of an alloy, wherein the alloy preferably contains a further metal being selected from group consisting of Cr, Ni, Mo and Co or at least two thereof, preferably Ni.

In order to achieve the preferred concentrations of constituents of the alloy, described above in the embodiments, the skilled person may vary the proportions of starting materials employed in the preparation process. The proportions of the starting materials might not be equal to the proportions of constituents of the product, due to net loss or gain during the preparation process.

Process for preparation of the alloy

The process for the preparation of the alloy preferably comprises the following steps:

    a) A vacuum induction melting step;
    b) A vacuum arc melting step.

In one embodiment of the invention, the process comprises two or more vacuum induction melting steps. In another embodiment of the invention, the process comprises two or more vacuum melting steps. In another embodiment of the invention, the process comprises two or more vacuum induction melting steps and two or more vacuum arc melting steps.

In preferred embodiments of the invention, the process further comprises one or more of the following steps:

    c) An electro-slag melting step
    d) A homogenisation step
    e) A cogging step
    f) A finish roll step
    g) A straightening step

In preferred embodiments, the process comprises a combination of the above steps selected from the list consisting of: c), d), e), f), g), c)+d), c)+e), c)+f), c)+g), d)+e), d)+f), d)+g), e)+f), e)+g), f)+g), c)+d)+e), c)+d)+f), c)+d)+g), c)+e)+f), c)+e)+g), c)+f)+g), d)+e)+f), d)+e)+g), d)+f)+g), e)+f)+g), d)+e)+f)+g), c)+e)+f)+g), c)+d)+f)+g), c)+d)+e)+g), c)+d)+e)+f) and c)+d)+e)+f)+g).

In one embodiment of the invention, one or more of the steps c)-g) is carried out two or more times.

In preferred vacuum induction melting steps, a material is heated by inducing an electric current in the material, preferably by electromagnetic induction. The pressure in the vacuum induction melting step is preferably below about 0.1 mbar, more preferably below about 0.01 mbar, most preferably below about 0.001 mbar. The vacuum induction melt step is preferably carried out in an oven, preferably with a low leak rate, preferably below about 0.1 mbar·l/s, more preferably below about 0.01 mbar·l/s, most preferably below about 0.001 mbar·l/s. The leak rate is preferably tested before the vacuum induction melting step by evacuating the oven, closing the valves of the oven, and measuring the rate of increase of pressure in the oven.

In one embodiment of the invention, the vacuum induction melting step is carried out in an inert atmosphere, preferably argon, preferably an atmosphere comprising at least about 90 wt. %, more preferably at least about 99 wt. %, most preferably at least about 99.9 wt. % of inert gas, preferably argon. In one aspect of this embodiment, the oven is evacuated and inert gas, preferably argon, introduced into the oven before melting. In one aspect of this embodiment, the pressure in the vacuum induction melting step is in the range from about 1 to about 200 mbar, preferably in the arrange from about 10 to about 150 mbar, most preferably in the range from about 20 to about 100 mbar.

In preferred vacuum arc melting steps, a material is heated by passing an electrical current through the material, preferably with an electrical power in the range from about 300 to about 1200 W/kg, more preferably in the range from about 400 to about 1000 W/kg, most preferably in the range from about 450 to about 900 W/kg, based on the mass of material heated. The pressure in the vacuum arc melting step is preferably below about 0.1 mbar, more preferably below about 0.01 mbar, most preferably below about 0.001 mbar. The vacuum arc melt step is preferably carried out in an oven, preferably with a low leak rate, preferably below about 0.1 mbar·l/s, more preferably below about 0.05 mbar·l/s, most preferably below about 0.01 mbar·l/s. The leak rate is preferably tested before the vacuum arc melting step by evacuating the oven, closing the valves of the oven, and measuring the rate of increase of pressure in the oven. In one embodiment of the invention, the vacuum arc melting step is carried out in an inert atmosphere, preferably argon, preferably an atmosphere comprising at least about 90 wt. %, more preferably at least about 99 wt. %, most preferably at least about 99.9 wt. % of inert gas, preferably argon. In one aspect of this embodiment, the oven is evacuated and inert gas, preferably argon, introduced into the oven before melting. In one aspect of this embodiment, the pressure in the vacuum arc melting step is in the range from about 0.001 to about 0.2 bar, preferably in the range from about 0.01 to about 0.15 bar, most preferably in the range from about 0.05 to about 0.1 bar.

Homogenisation steps according to the invention preferably allow reduction of inhomogeneity in a material, preferably by heating. In preferred homogenisation steps according to the invention, a material is heated to a temperature which is below its melting temperature, preferably below its incipient melting temperature. It is preferred that the material be homogenised for a duration in the range from about 10 min. to about 20 hours, more preferably in the range from about 3 hours to about 10 hours, most preferably in the range from about 5 hours to about 8 hours. Homogenisation is preferably carried out in a vacuum or in a gaseous atmosphere, preferably in a gaseous atmosphere. It is preferred that the homogenisation step be carried out close to atmospheric pressure, preferably in the range from about 0.5 to about 1.5 bar, more preferably in the range from about 0.8 to about 1.2 bar, most preferably in the range from about 0.9 to about 1.1 bar. In one preferred embodiment, the homogenisation step is carried out in air.

In preferred cogging steps according to the invention, the porosity or grain size or both of a material are reduced, preferably at elevated temperatures, preferably below the melting point of the material, preferably with the application of compressive force. Compressive forces may be applied locally or in a delocalised manner, preferably by one or more selected from the group consisting of: rolling, pressing, beating and turning. Where the material to be cogged has a mass below about 10 kg, preferably below about 8 kg, more preferably below about 5 kg, rolling is preferred. Where the material to be cogged has a mass above about 10 kg, preferably above about 20 kg, more preferably above about 30 kg, beating or turning is preferred. It is preferred that the smallest dimension of the material is reduced during the cogging process.

Preferred finish roll steps according to the invention reduce the smallest dimension of the material, preferably by passing the material through one or more pairs of rolls, preferably below the melting point of the material, more preferably below its incipient melting point. In one embodiment, the finish roll step reduces the porosity or grain size of the material, preferably both.

Straightening preferably reduces the physical curvature of the material, preferably so as to facilitate further grinding or machining steps. Straightening is preferably carried out by applying compressive force. The straightening step is preferably carried out below the melting point of the material, more preferably below its incipient melting point. In one embodiment, the process comprises a hot straightening step. In one embodiment, the process comprises a cold straightening step, preferably carried out at around ambient temperature. Cold straightening is preferably carried out at a temperature in the range from about 10 to about 100 °C, more preferably in the range from about 15 to about 80 °C, most preferably in the range from about 20 to about 50 °C.

Leads, wires and Medical Devices

In this text, reference is made variously to a coated or cladded wire, which comprises a wire core and a shell. The shell might be coated or cladded onto the core wire.

A preferred lead according to the invention comprises at least one proximal connector, at least one distal electrode and a flexible elongated conductor that is electrically connecting the electrode(s) to the connector(s). Preferably the elongated conductor is a coiled wire or a cable and comprises the alloy according to the invention.

A contribution to achieving at least one of the above mentioned objects is made by a wire comprising an alloy according to the invention, preferably having a thickness in the range from about 10 to about 50 μm, preferably in the range from about 15 to about 35 μm. In one embodiment, the wire further comprises silver metal.

In one embodiment, the lead comprises a silver core and an alloy according to the invention, preferably present as a shell surrounding the silver core.

A contribution to achieving at least one of the above mentioned objects is made by a lead comprising one or more wires according to the invention, preferably grouped into two or more cables, each cable comprising two or more wires according to the invention. In one embodiment, the cables have a thickness in the range from about 0.05 to about 0.5 mm, preferably in the range from about 0.1 to 0.4 mm.

A contribution to achieving at least one of the above mentioned problems is made by a medical device, preferably a pacemaker, comprising a lead according to the invention. A preferred pacemaker comprises:

- An implantable pulse generator;

- One or more leads according to the invention.

In one embodiment, the pacemaker comprises one or more pulsers.
In one embodiment, the pacemaker comprises one or more energy cells, preferably one or more electrical cells.
A process for the preparation of a wire comprises the steps:

a) Providing a tube of alloy according to the invention;
b) At least partially filling the tube with Ag to obtain a composite;
c) One or more drawing steps to reduce the diameter of the composite;
d) Optionally one or more annealing steps to soften the composite and facilitate drawing.

In one embodiment of the invention, the Ag content of the wire obtainable by the process is in the range from about 15 to about 50 wt. %, preferably in the range from about 17.5 to about 45.7 wt. %, more preferably in the range from about 28.7 to about 37.7 wt. %, based on the total weight of the wire.
In one embodiment, the diameter of the wire obtainable by the process is in the range from about 5 to about 50 $\mu$m, preferably in the range from about 15 to about 35 $\mu$m.
In one embodiment, the filling degree of silver in the wire obtainable by the process is in the range from about 15 % to about 41 %, preferably in the range from about 20 % to about 35 %, more preferably in the range from about 23 % to about 33 %.
It shall be understood that the method for manufacturing a passivated product, the passivated product, and the medical device comprising such passivated product according to the independent claims have similar and/or identical preferred embodiments, in particular, as defined in the dependent claims. It shall be understood further that a preferred embodiment of the invention can also be any combination of the dependent claims with the respective independent claim.
These and other aspects of the present invention will become apparent from and be elucidated with reference to the embodiments described hereinafter.

Description of the Drawings

The invention is now further illustrated using figures which are not to be considered as limiting the scope of the invention.

Figure 1 shows schematically a lead according to the invention.
Figure 2 shows schematically an apparatus for measuring fatigue resistance.
Figure 3 shows schematically a pacemaker comprising a lead according to the invention.
Figure 4 shows a cross sectional image of a wire of material according to example 2 (comparative).
Figure 5 shows a cross sectional image of a wire of material according to example 2 (comparative).
Figure 6 shows an analysis of elemental composition by energy dispersive x-ray spectroscopy of an inclusion in a wire of material according to example 2 (comparative).
Figure 7 shows a cross sectional image of a wire of material according to example 2 (comparative).
Figure 8 shows a cross sectional image of a wire of material according to example 2 (comparative).
Figure 9 shows a cross sectional image of a wire of material according to example 2a (comparative) with an Ag core.
Figure 10 shows a cross sectional image of a wire of material according to example 2a (comparative) with an Ag core.
Figure |11|, shows an analysis of elemental composition by energy dispersive x-ray spectroscopy of an inclusion in a wire of material according to example 2a (comparative) with an Ag core.
Figure 12 shows a plot of fatigue results for a wire of material according to example 1 (inventive) and a wire of material according to example 2 (comparative).
Figure 13 shows a plot of fatigue results for a wire of material according to example 1a (inventive) with an Ag core and a wire of material according to example 2a (comparative) with an Ag core.
Figure 14 shows a method for manufacturing a passivated product.

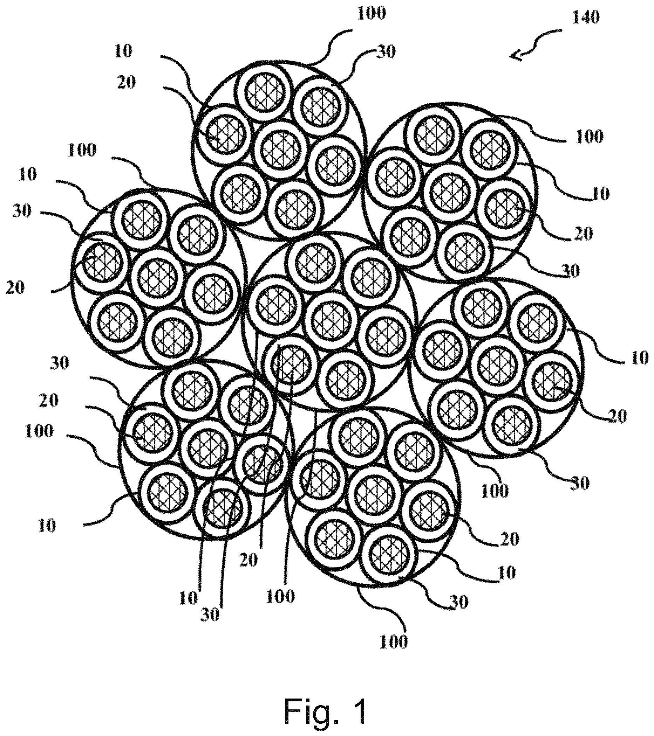

Figure 1 shows schematically a lead having a cable bundle 140, which comprises cables 100. In this example, the cables 100 each comprise 7 wires 10. Each wire comprises a first region 20 and a further region 30, wherein the first region 20 is interior to the region 30 along the length of the lead 140. The first region 20 is 41 area % of the cross sectional area the wire 10 and the further region is 59 area % of the cross sectional area of the wire 10, in each case based on the total cross sectional area of the wire 10. In this example, the first region 20 is silver. The further region 30 is a Cr, Ni, Mo and Co alloy as described above. In this example, the cable bundle 140 comprises 7 cables 100, each cable 100 comprising 7 wires 10. The invention is not limited to this arrangement. In particular, other arrangements of wires 10 in cables 100 and/or other arrangements of cable bundles 140 in leads are conceivable.

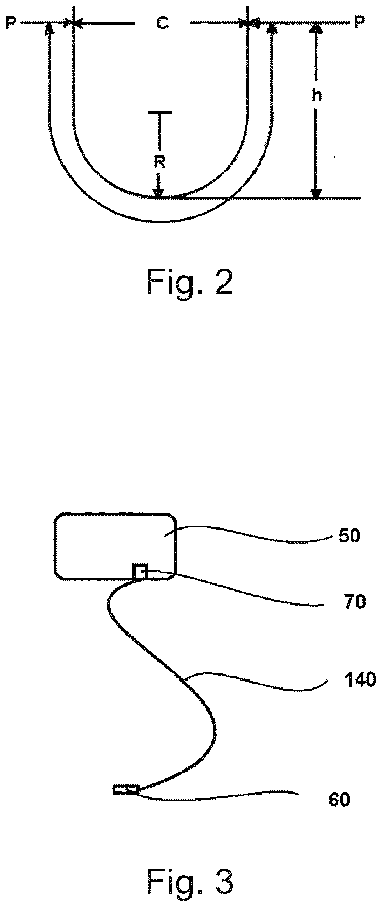

Figure 2 shows schematically an apparatus for measuring fatigue resistance.

Figure 3 shows schematically a pacemaker 50 with a pulse generator 70, and a lead 140 comprising an electrode 60. The lead 140 connects the pulse generator 70 and the heart tissue via the electrode 60.

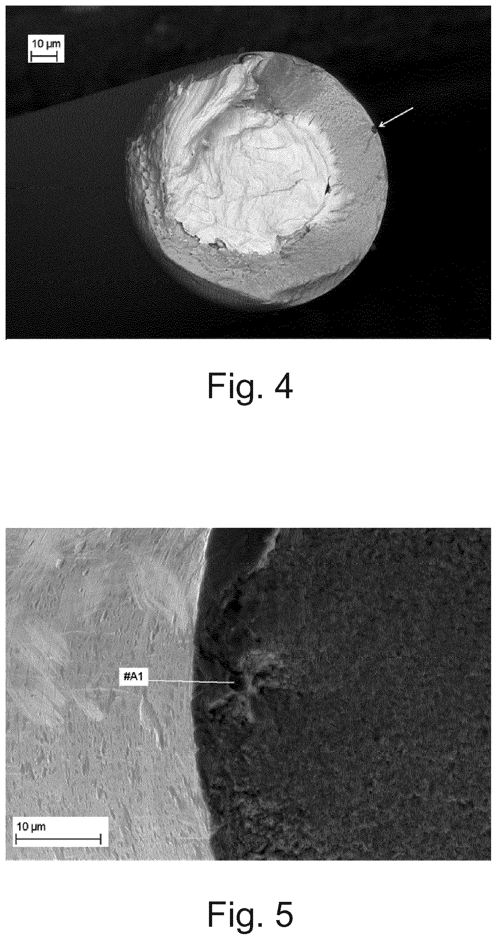

Figure 4 shows a cross sectional image of a wire of material according to example 2 (comparative) as observed by backscattered electron imaging according to the test method. A dark inclusion is indicated with an arrow.

Figure 5 shows a cross sectional image of a wire of material according to example 2 (comparative) as observed by backscattered electron imaging according to the test method. Figure 5 shows the same image as figure 4, but at higher magnification. A dark inclusion is indicated with the reference mark #A1.

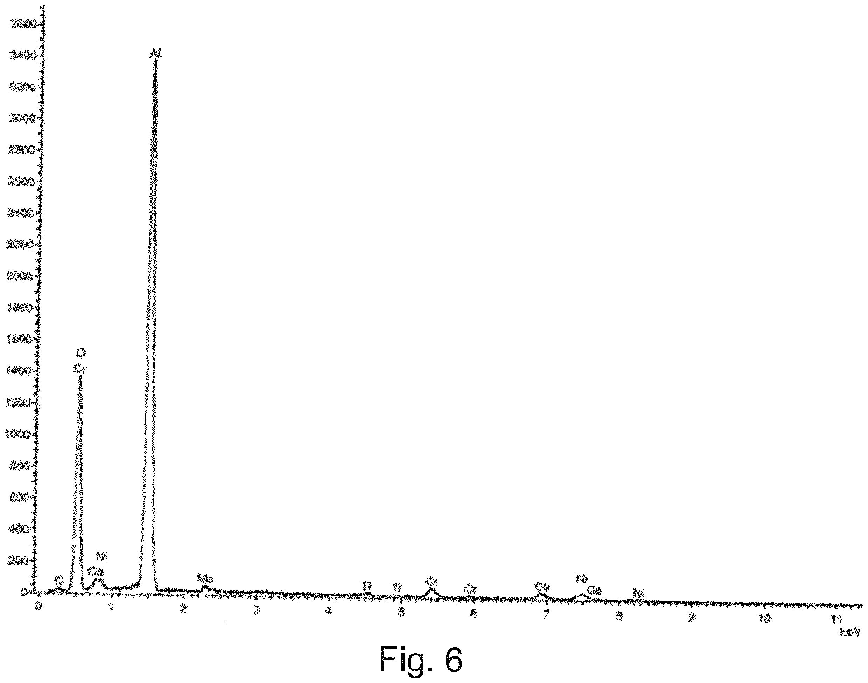

Figure 6 shows an analysis of elemental composition by energy dispersive x-ray spectroscopy according to the fracture surface analysis test method of the surface of an inclusion in a wire of material according to example 2 (comparative). The surface analysed is the inclusion indicated as #AI in figure 5. In particular, the analysis shows the presence of Al and Mg impurities and also of entities with a Cr-O bond.

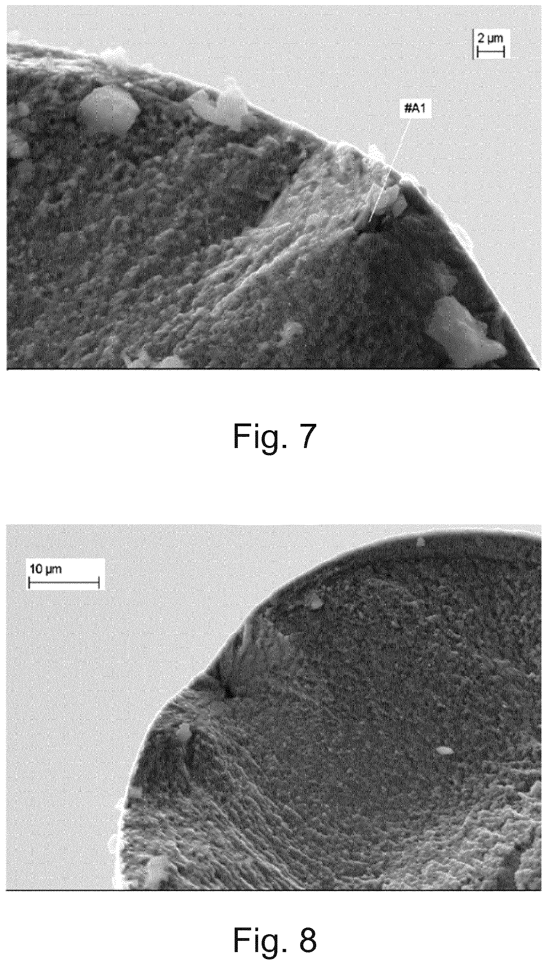

Figure 7 shows a cross sectional image of a wire of material according to example 2 (comparative) as observed by backscattered electron imaging according to the test method. A dark inclusion is indicated with the reference mark #A1.

Figure 8 shows a cross sectional image of a wire of material according to example 2 (comparative) as observed by backscattered electron imaging according to the test method. The surface shown in figure 8 is taken from the same slice as that of figure 7.

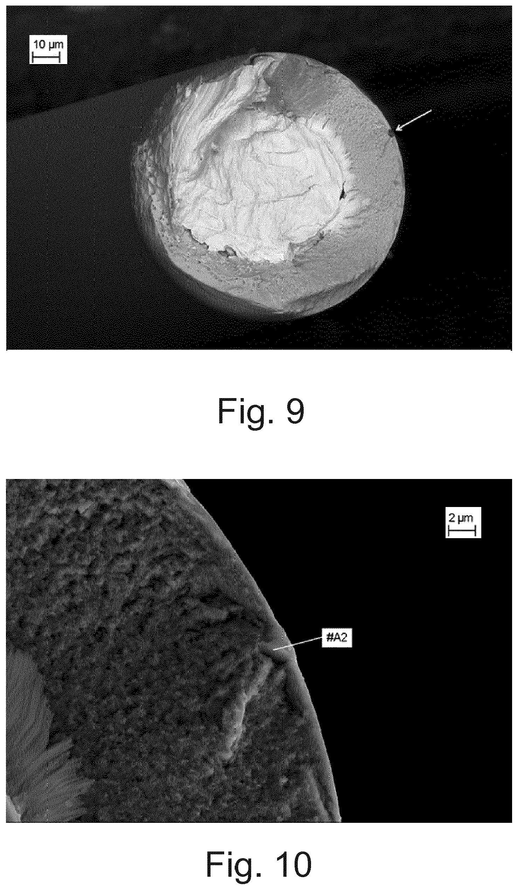

Figure 9 shows a cross sectional image of a wire of material according to example 2a (comparative) with an Ag core, as observed by backscattered electron imaging according to the test method. A dark inclusion is indicated with an arrow.

Figure 10 shows a cross sectional image of a wire of material according to example 2a (comparative) with an Ag core, as observed by backscattered electron imaging according to the test method. Figure 10 shows the same image as figure 9, but at higher magnification. A dark inclusion is indicated with the reference mark #A2.

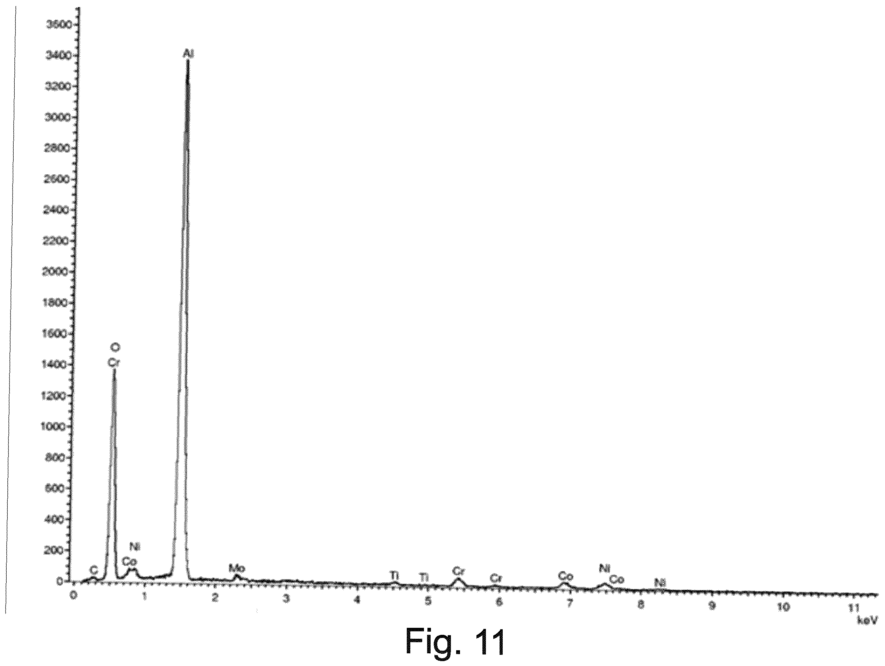

Figure 11 shows an analysis of elemental composition by energy dispersive x-ray spectroscopy according to the fracture surface analysis test method of the surface of an inclusion in a wire of material according to example 2a (comparative) with an Ag core. The surface analysed is the inclusion indicated as #A2 in figure 10. In particular, the analysis shows the presence of Al impurities and also of entities with a Cr-O bond.

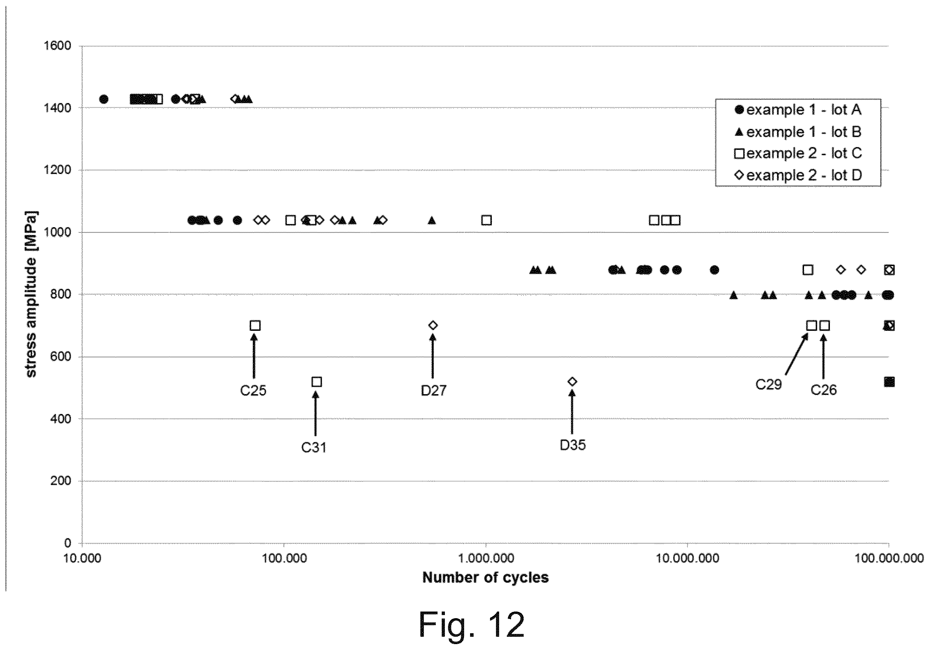

Figure 12 shows a plot of fatigue results for a wire of material according to example 1 (inventive) and a wire of material according to example 2 (comparative). For example 1 (inventive), results are shown for 2 lots, lot A as represented by a solid circle and lot B as represented by a solid triangle. For example 2 (comparative), results are shown for 2 lots, lot C as represented by a hollow square and lot D as represented by a hollow diamond. The number of cycles before failure is shown as dependent on the stress amplitude applied in the test. Outliers, which performed poorly are indicated with arrows.

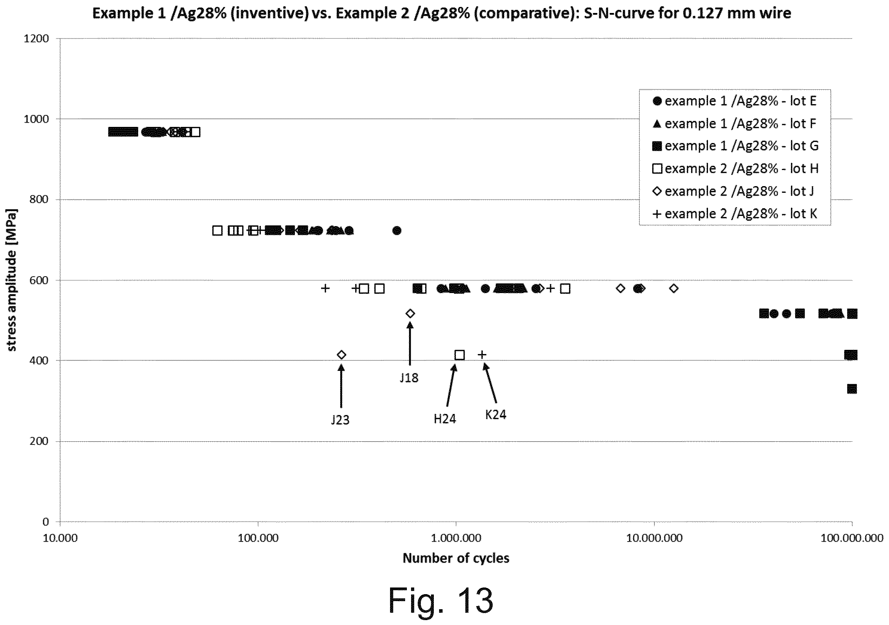

Figure 13 shows a plot of fatigue results for a wire of material according to example 1a (inventive) with an Ag core and a wire of material according to example 2a (comparative) with an Ag core. For example 1a (inventive), results are shown for 3 lots, lot E as represented by a solid circle, lot F as represented by a solid triangle and lot G as represented by a solid square. For example 2a (comparative), results are shown for 3 lots, lot H as represented by a hollow square, lot J as represented by a hollow diamond and lot K as represented by a cross. The number of cycles before failure is shown as dependent on the stress amplitude applied in the test. Outliers, which performed poorly are indicated with arrows.

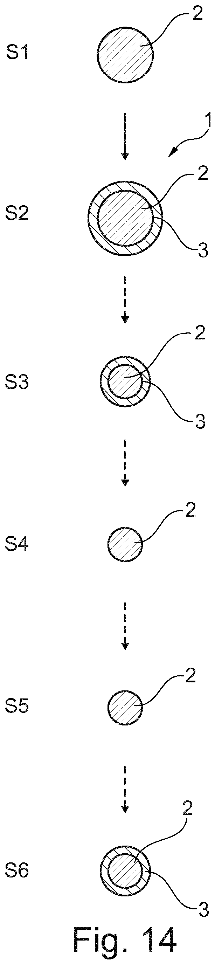

Figure 14 shows a method for manufacturing a passivated product 1. The method for manufacturing the passivated product 1 comprises the following steps:

In step S1, providing a raw product 2.
The raw product 2 is a wire at a diameter of 2 mm and a length of 15 m. The raw product 2 is made at least partially of an alloy comprising the following alloy components: Cr in the range from about 10 to about 30 wt. %; Ni in the range from about 20 to about 50 wt. %; Mo in the range from about 2 to about 20 wt. %; and Co in the range from about 10 to about 50 wt. %. The Al content of the Cr, Ni, Mo and Co alloy is less than about 0.01 wt. % and each wt. % is based on the total weight of the alloy.
Before passivation in step S2, the raw product 2 is cleaned so that any residual lubricants used are removed. This can be accomplished for example by ultrasonic cleaning in a mild alkaline cleaning agent (Deconex-12PA) at 40-60 °C for 15 min (in a batch process). The raw product 2 is then rinsed with DI water and optional wiped with IPA tissues.

In step S2, passivating a surface of the raw product 2 at least partially to provide a passivated product 1 with a passivation coating 3.
The passivation is completed with a low temperature nitric acid solution (Nitric 2). Specifically, this is done at 20 °C in an aqueous solution with 45 vol.-% nitric acid for 45 min. The passivation coating 3 is provided directly on the metallic surface of the alloy, is inert in conditions during wire drawing and has a thickness in the range of 10 nm to 50 $\mu$m. After removal from the above mentioned passivation solution, the passivated product 1 is thoroughly rinsed with DI water. To ensure the rinse is completed properly, the electrical conductivity of the rinse water after passing though the passivated product 1 should be less than 15 $\mu$S/cm.

In step S3, an optional wire drawing to a diameter of 0.4 mm is conducted.
The passivation coating 3 protects the alloy surface from direct contact with the drawing tool (drawing die). The overall cold work in this example is 96 %. The diameter of the semi-finished part should match with the desired cold work to meet mechanical properties.

In step S4, if an (intermediate) heat treatment is required, the passivation coating 3 needs to be removed (mechanical and/or chemical).

In step S5, the heat treatment follows.

In step S6, the passivation coating 3 is re-applied after the heat treatment.

The passivation coating 3 on the wire may be renewed e.g. by above mentioned process from spool to spool including cleaning and passivation. Specifically, this can be done in-line (from spool to spool) at for example a speed of 5-10 m/min in a sequences of baths with a mild alkaline cleaning agent at 80 °C with ultrasonic followed by a rinse with DI water, followed by a IPA cleaning.

Test Methods

Alloy composition

For a quantitative chemical analysis of the alloy, the following methods are used:

a) the main components of the alloy (Co, Cr, Ni, Mo) are measured by X-ray fluorescence XRF using the XRF Lab Report - S8 TIGER from the company BRUKER (Bruker AXS GmbH Ostliche Rheinbrückenstr. 49, 76187 Karlsruhe, Germany)
b) Trace elements present in the alloy (Mn, P, Si, Fe, Ti, Al, B, Mg, Ca, Ce, Ti) are measured by glow discharge mass spectrometry (GDMS) using the ASTRUM from Nu Instruments (Nu Instruments Limited, Unit 74, Clywedog Road South, Wrexham, LL13 9XS UK.)
c) Gas or non-metallic components in the alloy (H, O, C, N, S) are measured by carrier-gas hot extraction using the ONH836 from LECO (LECO Corporation, 3000 Lakeview Avenue, St. Joseph, Michigan 49085)

Leak rate

The leak rate of the furnace chamber is measured using the following procedure:

The Vacuum furnace chamber is evacuated to the required pressure by a vacuum pumping station. When the required pressure is reached, the pressure valve between the vacuum furnace chamber and the vacuum pumping station is closed. The pressure increase of the vacuum furnace chamber over a given length of time defines the leak rate of the equipment.

Fatigue resistance

Rotating beam fatigue testing was carried out using Valley Instruments model # 100 test machine (Figure 2) according to Valley Instruments Wire Fatigue Tester Model # 100 user manual (Valley Instruments (Division of Positool Technologies, Inc.), Brunswick, Ohio, USA. Fatigue Tester Model 100 Manual). The equipment consists of a synchronous motor rotating at 3600 rpm. For each test of a wire specimen, a sample having a predefined length is fixed in a custom fine-wire collet at one end, looped through a complete 180 degree turn and is placed at the other end in a low-friction bushing in which it is free to rotate. The synchronous motor of the test device is directly clocked by a counter where the number of cycles is shown in a LCD-display. The fatigue testers are equipped with a sensor to detect the wire fracture which automatically stops the timer, means the display of the timer shows the number of cycles until failure. If no fracture occurs within 100 Million cycles, the test is stopped.
Valley Instruments Wire Fatigue Tester Model # 100 user manual (Valley Instruments (Division of Positool Technologies, Inc.), Brunswick, Ohio, USA. Fatigue Tester Model 100 Manual) describes that a loop, formed by an elastic length held so that the axes of the specimen at the point of retention are exactly parallel, assumes a shape in which:

(1) The length of the loop is 2.19 times the base,
(2) The height of the arch is always 0.835 times the base,
(3) The minimum radius of the curvature occurs at the apex of the arch and is exactly 0.417 times the base, and
(4) The bending stress at the point of minimum curvature bears a simple reciprocal linear relationship to any of the four physical dimensions (length, height, base, and minimum curvature).

The following formulas express the exact relationship:

$$C = 1.198 * E * d / S$$

$$h = 0.835 * C$$

$$L = 2.19 * C$$

$$R = 0.417 * C$$

$$P = 0.141 * E * d^4 / C^2$$

Nomenclature:

C = chuck to bushing distance
d = diameter of wire
h = height of loop
E = modulus of elasticity
L = length of wire external to chucks
R = minimum of radius of curvature
S = bending stress
P = bushing load or lateral force at the chuck

With the above listed formula, the bending stress S (at the peak of the loop) can be calculated by the following equation:

$$S = 1.198 * E * d / C$$

The machine set-up involves calculating the desired sample length and center distance using the modulus of elasticity of the material and equations developed by Valley Instruments Company (user manual).

Microscopic Inspection Method for Micro-cleanliness

Definition: Inclusions are defined as internal flaws or contaminations (such as nitrides or oxides) within the billet or rod from which the wire or tube is produced. The transverse inclusion size is defined as the largest dimension of an internal flaw measured on transverse cross-sections of the billet, rod or wire. The longitudinal inclusion size is defined as the largest dimension of an internal flaw measured on longitudinal cross-sections of the billet, rod or wire. A cross-section diametral line is defined as any line within the cross-section having a length equal to or greater than 95% of the true cross-section diameter.

General Test procedure:

a) Sectioning

For each material lot, the billet, rod or wire is to be sectioned at each end so that there are an equal number of cross sections sampled at the one end as there are samples at the other end (number of samples taken from each end shall differ by no more than one). The total number of cross sections samples depends on the diameter of the billet, rod or wire and is specified in Table 1. The length of each cross section is to be less than its diameter.

b) Imaging

For each billet, rod or solid wire cross-section, non-overlapping images are to be taken at 500X magnification along diametral lines so that the total examined area per sample is at least 1.77 mm$^2$. A cross-section diametral line is defined as any line within the cross-section having a length equal to or greater than 95% of the true cross-section diameter. Angular separation between two diametral lines on a cross- section shall be a minimum of 60 degrees. The number of images and the number of diametral lines depends on the diameter of the billet, rod or wire and is specified in Table 1.

The total number of images is shown in Table 1 and was calculated based on the number of images per sample and the number of samples.

c) Measurement

Each of the images is to be inspected to detect the presence of inclusions or strings of inclusions that exceed a size of 3.0 $\mu$m in their largest dimension. The image inspection may be accomplished either by manual examination or by automated scanning.

**Table 1**

| cross section diameter of billet, rod or wire | | Number of diametral lines per section (no requirement for tube samples) | Number of images per section | Number of cross-sections | | Total images per lot | |
|---|---|---|---|---|---|---|---|
| equal to or greater than [mm] | but no greater than [mm] | | | transverse | longitudinal | transverse | longitudinal |
| 2.54 | 3.80 | 5 | 40 | 12 | 12 | 480 | 480 |
| 3.81 | 5.71 | 3 | 40 | 12 | 12 | 480 | 480 |
| 5.72 | 11.42 | 2 | 40 | 12 | 12 | 480 | 480 |
| 11.43 | 13.96 | 1 | 40 | 12 | 12 | 480 | 480 |
| 13.97 | 17.14 | 1 | 48 | 10 | 10 | 480 | 480 |
| 17.15 | 21.58 | 1 | 60 | 8 | 8 | 480 | 480 |
| 21.59 | 27.93 | 1 | 80 | 6 | 6 | 480 | 480 |
| 27.94 | 33.01 | 1 | 96 | 5 | 5 | 480 | 480 |
| 33.02 | 43.17 | 1 | 120 | 4 | 4 | 480 | 480 |
| 43.18 | 57.14 | 1 | 160 | 3 | 3 | 480 | 480 |

Fracture surface analysis of wire samples

The test method to analyse fracture surfaces of fatigue tested samples was Scanning electron microscopy (SEM). A Zeiss Ultra 55 Gemini was used for the sample analysis of the present invention and comparative samples.

Two imaging modes were used to analyse and illustrate the tested samples.

a) SE: the detection of secondary electrons (SE) results in images with a well-defined, three-dimensional appearance. The surface topography can be illustrated in high resolution. Figures 5, 7, 8 and 10 are secondary electron images.
b) BSE: backscatter electrons (BSE) are used to detect contrast between areas with different chemical compositions. Heavy elements (high atomic number) backscatter electrons more strongly than light elements (low atomic number), and thus appear brighter in the image. Figures 4 and 9 are BSE images.

Energy-dispersive X-ray spectroscopy (EDS, EDX) was used for the elemental analysis of features (inclusions/particles) found on the fatigue resistance test samples. A high-energy beam of electrons is focused onto the location of the sample being analysed. This leads to the emission of characteristic X-rays which allows the elemental composition of the feature (inclusions/particles) to be measured. Figures 6 and 11 show EDX scans.

Examples

The MP35N heats were VIM-VAR melted, to minimize the impurity content and to obtain a sound ingot with good chemical uniformity and metallurgical properties. The chemistry of representative heats: Heat 1, Heat 2 and Heat 3 are listed in Table 3. The table also provides the chemistry of a VIM-VAR melted, commercially available MP35N alloy and for reference the chemical requirements per ASTM F562-13, a standard specification for wrought MP35N alloy. The major constituents of MP35N alloy are Co, Ni, Cr and Mo. The new alloy heats were melted in 2 steps. The first melting step was Vacuum Induction Melting (VIM). The VIM furnace consists of a water cooled vacuum melt chamber, an oxide ceramic crucible held in a cylindrical induction heating coil inside the melt chamber, an AC electric power supply, a vacuum pumping system, a raw material adding chamber and a cylindrical metal mold held below and offset from the crucible-induction coil assembly. The vacuum melt chamber, raw material adding chamber and vacuum pumping system are separated by isolation valves. The induction heating coil is water cooled. Electric current from the power supply passes through the induction heating coil creating a magnetic field inside the furnace. The magnetic field induces eddy currents inside the raw materials causing Joule heating. Joule heating raises the temperature of the raw materials to above their melting point. The magnetic field mixes the liquid raw materials to make a homogeneous alloy. The crucible is tilted to pour the liquid alloy from the crucible into the mold. The alloy cools to a solid in the mold under vacuum and is removed from the furnace. The alloy ingot is removed from the mold and it is prepared for re-melting.
For the example heats, 136 kilograms of elemental raw materials were placed in the furnace in proportions calculated to make the aim chemistry. The VIM furnace was closed and pumped down to ≤ 0.00001 bar. A leak-up rate was measured after reaching the desired vacuum pressure level to ensure a vacuum tight furnace. The leak-up rate was ≤ 0.00001 bar/min. Electric power was applied to the induction heating coil. Once the melt was in progress, the vacuum level was recorded at specified intervals to monitor the progress of melting and the mixing and reaction of all of the raw materials. When the reactions ceased as indicated by a constant vacuum pressure level, the heat was poured into a 152.4 mm diameter cylindrical mold.
Each heat was subsequently re-melted by a Vacuum Arc Re-melting (VAR) process to make an 203.2 mm diameter ingot. The VAR furnace consists of water cooled vacuum chamber, a 203.2 mm diameter water cooled copper crucible, a direct current electric power supply, a vacuum pumping system, isolation valves and a computer based electrical system to monitor and control the application of current to the electrode inside the vacuum chamber. The furnace was pumped down to ≤ 0.000006 bar before carrying out the leak-up rate test. A leak rate of ≤ 0.000006 bar /min was obtained. The electrode was moved to a close proximity to the bottom of the crucible. Electric power was applied at a level to cause an electric arc to be struck between the crucible bottom and the alloy electrode. The electric arc causes the electrode to melt and drip into the bottom of the crucible creating a liquid metal pool that solidifies as the arc moves away from the molten pool. The process was continued at a controlled rate until the electrode was consumed. The power was turned off and the ingot was cooled under vacuum. The ingot was removed from the furnace for processing to product.
The as-cast ingot was charged into a gas-fired front opening box furnace with ambient air atmosphere. The furnace was preset to a temperature of 815°C. Upon equilibration of furnace temperature, the ingot was held for additional 4 hours prior to raising the furnace temperature. The ingot was then heated to 1177°C at a heating rate of 200 K per hour. The ingot was held for 7 hours at 1177°C for homogenization. After homogenization, the ingot was hot rolled from 203 mm to 137 mm round cornered square (RCS) billet using a 559 mm diameter Morgenshammer Mill operating at ambient temperature. The Morgenshammer Mill is a manually operated tilt table mill with 3 high rolls allowing heavy bar to be rolled alternately between the bottom and middle roll and the top and middle roll. After hot rolling the RCS billet was air cooled, abrasively ground by hand to remove surface imperfections and cut to square the ends. The billet was reheated and hot rolled to 51 mm RCS at 1177°C on the 559 mm Morgenshammer Mill. The RCS was cut to shorter lengths of final rolling on a hand operated 406 mm diameter Morgenshammer Mill with 3 high rolls. All bar manipulation on this mill is done by hand at floor level. The RCS was reheated at 1177°C and rolled to 33.4 mm round bars and air cooled to ambient temperature. The rolled bars were then reheated to 1038°C and held for 30 minutes for hot rotary straightening. After straightening, the bars were air cooled to room temperature. The bars were rough centerless ground, ultrasonic tested for voids and then centerless ground to final size.

For manufacturing of clad-wires, the grinded bars were gun-drilled to produce hollows for subsequent tube drawing. Tubes were filled with Ag-rods and cold-drawn using diamond dies and mineral oil. For a final wire diameter of 127 $\mu$m, the last intermediate annealing was carried out at a wire diameter of 157.5 $\mu$m at 900 - 950 °C in Argon atmosphere. From the last intermediate annealing until the final diameter of the wire, 35% cold-work were applied. Three wire lots were manufactured having UTS values of 1456, 1469 and 1474 MPa. For bare wire, the bars were further hot-rolled to 0.2 inch outer diameter followed by cold-drawing. For 102 $\mu$m final size wire, the last intermediate annealing was carried out at a wire diameter of 122 $\mu$m at 1100 °C in Argon atmosphere to apply 30% cold-work to the final size. Two wire lots were manufactured having UTS values of 1870 and 1875 MPa. The wires of inventive example 1 (Lots A & B) and the cladded wires of inventive example 1a (Lots E, F & G) were made using the alloy of Heat 1 in table 3. The wires of comparative example 2 (lots C & D) and the cladded wires of comparative example 2a (lots H, J & K) were made from the alloy of the commercial heat in table 3 obtained from Fort Wayne Metals, Inc., USA under the trade name 35 NLT®.

**Table 2**

| Material | Example 1 (inventive) | | Example 1a with 28 % Ag (inventive) | | |
|---|---|---|---|---|---|
| Batch | Lot A | Lot B | Lot E | Lot F | Lot G |
| UTS [MPa] | 1870 | 1875 | 1456 | 1469 | 1474 |
| YM [GPa] | 190 | 191 | 121 | 121 | 122 |
| Elongation [%] | 2.8 | 2.9 | 2.2 | 2.3 | 2.3 |

The processed alloy was also obtainable from SAES Smart Materials, Inc. Alloys for the further examples were acquired from SAES Smart Materials, Inc.

**Table 3**

| Element | Heat 1 | Heat 2 | Heat 3 | Commercial Heat | ASTM F-562-13 |
|---|---|---|---|---|---|
| | Wt. % | Wt. % | Wt. % | Wt. % | Wt. % |
| C | 0.0039 | 0.0091 | 0.0106 | 0.005 | < 0.0250 |
| B | 0.000065 | 0.000067 | 0.000008 | 0.01 | < 0.015 |
| P | 0.00018 | 0.000095 | 0.000056 | 0.001 | < 0.015 |
| S | 0.00056 | 0.00026 | 0.00036 | 0.001 | < 0.010 |
| Mn | 0.00028 | 0.00021 | 0.00013 | 0.017 | < 0.15 |
| Si | 0.0042 | 0.0053 | 0.0061 | 0.034 | < 0.15 |
| Al | 0.00023 | 0.00054 | 0.00043 | 0.023 | NA |
| Mg | <0.000001 | 0.000003 | 0.000005 | 0.001 | NA |
| Ca | <0.000005 | <0.000005 | <0.000005 | NA | NA |
| Ce | <0.000001 | <0.000001 | <0.000001 | NA | NA |
| Fe | 0.021 | 0.023 | 0.023 | 0.08 | < 1 |
| Ti | 0.00017 | 0.000038 | 0.000023 | 0.001 | < 1 |
| O | 0.0085 | 0.0056 | 0.0035 | 0.0021 | NA |
| N | 0.0022 | 0.0009 | 0.0007 | 0.0022 | NA |
| Cr | 19.6 | 19.7 | 20 | 20.62 | 19 - 21 |
| Ni | 35.7 | 34.8 | 34.9 | 34.91 | 33 - 37 |
| Mo | 10 | 9.93 | 9.7 | 9.47 | 9 - 10.5 |

(continued)

| Element | Heat 1 | Heat 2 | Heat 3 | Commercial Heat | ASTM F-562-13 |
|---------|--------|--------|--------|-----------------|---------------|
| | Wt. % | Wt. % | Wt. % | Wt. % | Wt. % |
| Co | balance | balance | balance | balance | balance |

Microscopic Inspection for Microcleanliness of the alloy

The microscopic inspection for microcleanliness of the inventive alloy (example 1 and example 1a with an Ag core) and of the comparative alloy (example 2 and example 2a with an Ag core) was carried out according to the procedure and test method described above. Of 4 rods with an outer diameter of 31.75 mm, 5 transverse and 5 longitudinal sections were taken according to table 1 and metallographically prepared. The sections included a continuous plane from two surface locations and through the approximated center of the bar. The metallographically prepared sections were examined in the as-polished condition by scanning electron microscopy (SEM) using backscattered electron imaging (BEI). In BEI, the brightness of sample features is proportional to the atomic weight of the elements constituting those features. Thus, in BEI, present inclusions consisting of heavier elements than the surrounding matrix material appear brighter than the matrix material. Inclusions consisting of lighter elements than the surrounding matrix material appear darker than the matrix material. Since nonmetallic inclusions (e.g. oxide or nitride inclusions) consist of lighter elements than the alloys of example 1 and example 2, in BEI these ceramic inclusions appear darker than the surrounding matrix material. Images were acquired at a magnification of 500X along a diametral line extending across the entire bar. Analysis of features darker and brighter than the background was conducted on the images using image analysis software to determine the maximum dimension for each detected feature. The largest dimension and area were recorded for each individual feature. The inclusions were categorized by largest dimension into 1 $\mu$m groups up to 14 $\mu$m. The total area of the dark and bright features was also calculated. Inclusions greater than 14 $\mu$m were also counted. Features smaller than 3.0 $\mu$m were not included in the measurements.

For each section, forty-eight fields of view were evaluated. For each direction, longitudinal and transverse, 480 images with a total area of 22.6 mm$^2$ were evaluated. The samples contained features that appeared darker and brighter than the bulk material using backscattered electron imaging. The darker features have a lower mean atomic number than the background and the brighter features have a higher mean atomic number than the background. Results of the inclusion analysis of example 1 are shown in tables 4-6. Results of the inclusion analysis of example 2 are shown in tables 7-10. Image fields showing typical dark (ceramic) inclusions are shown in Figures 4,5, 7-10.

Alloy of the present invention (example 1):

### TABLE 4 - FEATURE COUNT TOTALS / EXAMPLE 1

| Largest Dimension [$\mu$m] | | Number of Features | | | |
|---|---|---|---|---|---|
| | | Longitudinal | | Transverse | |
| | | Dark | Bright | Dark | Bright |
| 3.0 | - 3.9 | 15 | 0 | 14 | 0 |
| 4.0 | - 4.9 | 4 | 0 | 2 | 0 |
| 5.0 | - 5.9 | 2 | 0 | 1 | 0 |
| 6.0 | - 6.9 | 0 | 0 | 0 | 0 |
| 7.0 | - 7.9 | 0 | 0 | 0 | 0 |
| 8.0 | - 8.9 | 0 | 0 | 0 | 0 |
| 9.0 | - 9.9 | 0 | 0 | 0 | 0 |
| 10.0 | - 10.9 | 0 | 0 | 0 | 0 |
| 11.0 | - 11.9 | 0 | 0 | 0 | 0 |
| 12.0 | - 12.9 | 0 | 0 | 0 | 0 |

(continued)

| | Number of Features | | | |
|---|---|---|---|---|
| Largest Dimension [μm] | Longitudinal | | Transverse | |
| | Dark | Bright | Dark | Bright |
| 13.0 - 13.9 | 0 | 0 | 0 | 0 |
| 14.0 - 14.9 | 0 | 0 | 0 | 0 |
| >14.9 | 0 | 0 | 0 | 0 |
| | | | | |
| Total | 21 | 0 | 17 | 0 |

**TABLE 5 - TOTAL INCLUSION AREA MEASUREMENTS FOR EXAMPLE 1**

| | Area of Inclusions > 3 μm in Length for Examination Region | | | | | |
|---|---|---|---|---|---|---|
| | Darker | | Brighter | | All | |
| Sample | Total | Percent of Total Area | Total | Percent of Total Area | Total | Percent of Total Area |
| | [μm$^2$] | [%] | [μm$^2$] | [%] | [μm$^2$] | [%] |
| Longitudinal | 121 | 0.0006 | 0 | 0.0000 | 121 | 0.0006 |
| Transverse | 97 | 0.0005 | 0 | 0.0000 | 97 | 0.0005 |

**TABLE 6 - LONGEST DARK FEATURES FOR EXAMPLE 1**

| | Feature Dimensions, [μm] | | |
|---|---|---|---|
| Number | Length | Breadth | Direction |
| 1 | 5.6 | 3.0 | Longitudinal |
| 2 | 5.4 | 3.7 | Longitudinal |
| 3 | 5.4 | 2.9 | Longitudinal |
| 4 | 4.9 | 2.2 | Longitudinal |
| 5 | 4.7 | 3.6 | Longitudinal |
| 6 | 4.6 | 1.9 | Longitudinal |
| 7 | 4.5 | 2.7 | Longitudinal |
| 8 | 4.5 | 2.4 | Longitudinal |
| 9 | 4.1 | 2.4 | Longitudinal |
| 10 | 3.9 | 3.0 | Longitudinal |

example 2 (comparative):

**TABLE 7 - FEATURE COUNT TOTALS / BARS 1-10 / ALL SAMPLES**

| | Number of Features | | | |
|---|---|---|---|---|
| Largest Dimension [μm] | Longitudinal | | Transverse | |
| | Dark | Bright | Dark | Bright |
| 3.0 - 3.9 | 25 | 21 | 6 | 46 |

(continued)

| Largest Dimension [μm] | Number of Features | | | |
|---|---|---|---|---|
| | Longitudinal | | Transverse | |
| | Dark | Bright | Dark | Bright |
| 4.0 - 4.9 | 19 | 7 | 3 | 11 |
| 5.0 - 5.9 | 7 | 1 | 1 | 1 |
| 6.0 - 6.9 | 6 | - | - | 1 |
| 7.0 - 7.9 | 7 | - | - | - |
| 8.0 - 8.9 | 4 | - | - | - |
| 9.0 - 9.9 | 1 | - | - | - |
| 10.0 - 10.9 | 2 | - | - | - |
| 11.0 - 11.9 | 2 | - | - | - |
| 12.0 - 12.9 | - | - | - | - |
| 13.0 - 13.9 | - | - | - | - |
| 14.0 - 14.9 | - | - | - | - |
| >14.9 | 4 | - | - | - |
| | | | | |
| Total | 77 | 29 | 10 | 59 |

### TABLE 8 - TOTAL INCLUSION AREA MEASUREMENTS FOR EXAMPLE 2

| Sample | Area of Inclusions > 3 μm in Length for Examination Region | | | | | |
|---|---|---|---|---|---|---|
| | Darker | | Brighter | | All | |
| | Total | Percent of Total Area | Total | Percent of Total Area | Total | Percent of Total Area |
| | [μm$^2$] | [%] | [μm$^2$] | [%] | [μm$^2$] | [%] |
| Longitudinal | 409 | 0.0018 | 75 | 0.0003 | 484 | 0.0021 |
| Transverse | 69 | 0.0003 | 152 | 0.0007 | 221 | 0.0010 |

### TABLE 9 - LONGEST DARK FEATURES FOR EXAMPLE 2

| Number | Feature Dimensions, [μm] | | |
|---|---|---|---|
| | Length | Breadth | Direction |
| 1 | 33.4 | 1.9 | Longitudinal |
| 2 | 18.9 | 1.6 | Longitudinal |
| 3 | 17.8 | 2.3 | Longitudinal |
| 4 | 15.4 | 1.4 | Longitudinal |
| 5 | 11.8 | 1.0 | Longitudinal |
| 6 | 11.1 | 1.1 | Longitudinal |
| 7 | 10.6 | 1.0 | Longitudinal |
| 8 | 10.3 | 1.8 | Longitudinal |
| 9 | 9.5 | 1.5 | Longitudinal |

(continued)

| | Feature Dimensions, [$\mu$m] | | |
|---|---|---|---|
| Number | Length | Breadth | Direction |
| 10 | 8.9 | 2.2 | Longitudinal |

**TABLE 10 - LONGEST BRIGHT FEATURES FOR EXAMPLE 2**

| | Feature Dimensions, [$\mu$m] | | |
|---|---|---|---|
| Number | Length | Breadth | Direction |
| 1 | 6.0 | 1.6 | Transverse |
| 2 | 5.6 | 2.8 | Longitudinal |
| 3 | 5.1 | 1.8 | Transverse |
| 4 | 4.9 | 2.3 | Transverse |
| 5 | 1.9 | 1.8 | Transverse |
| 6 | 1.0 | 1.8 | Longitudinal |
| 7 | 4.6 | 1.3 | Transverse |
| 8 | 4.5 | 1.2 | Transverse |
| 9 | 4.4 | 1.6 | Longitudinal |
| 10 | 4.4 | 0.9 | Transverse |

According to Table 8 of example 2 (comparative), the total area of dark inclusions found is 478 $\mu$m$^2$ (409 $\mu$m$^2$ in longitudinal direction and 69 $\mu$m$^2$ in transverse direction). According to Table 5 of example 1 (inventive), the total area of dark inclusions found is only 218 $\mu$m$^2$ (121 $\mu$m$^2$ in longitudinal direction and 97 $\mu$m$^2$ in transverse direction). So the amount of dark inclusions (Percent of total area) in example 1 (inventive) is only 4.8 ppm (0.00048 %) while in example 2 (comparative) the amount of dark inclusions is 11 ppm (0.0011 %). In terms of inclusions (micro-cleanliness) this means that example 1 (inventive) is more than 2 times cleaner than example 2 (comparative).

Fatigue Test Results

Two lots of wire of example 1 (dia. 102 $\mu$m) were tested against two lots of wire or example 2 (same diameter - 102 $\mu$m) having comparable mechanical properties (UTS of 1862 - 1875 MPa).

**Table 11**

| Material | Example 1 (inventive) | | Example 2 (comparative) | |
|---|---|---|---|---|
| Batch | Lot A | Lot B | Lot C (Fig. 5 & 6) | Lot D |
| UTS [MPa] | 1870 | 1875 | 1862 | 1871 |
| YM [GPa] | 190 | 191 | 190 | 190 |
| Elongation [%] | 2.8 | 2.9 | 2.7 | 2.8 |

At an applied stress of 700 MPa, the wire of all four lots reached the fatigue endurance limit, means the wire does not fail and tests are stopped after 100 Million cycles. While the wire of example 1 showed no outliers at 700 MPa and below, 4 samples of example 2 failed at less than 2.7 Million cycles and two other samples ran 40 - 50 Million cycles. All other samples tested at an applied stress of 700 MPa and below survived 100 Million cycles without rupture. For Example 2 wire lot C, sample C25 tested at an applied stress of 700 MPa broke after only 71,790 cycles and sample C31 tested at an applied stress of 520 MPa broke after only 145,260 cycles. Sample C26 tested at an applied stress of 700 MPa broke after 47,547,540 cycles and sample C29 tested at an applied stress of 700 MPa broke after 41,282,990 cycles. For example 2 wire lot D, sample D27 tested at an applied stress of 700 MPa broke after only 549,227 cycles and sample D35 tested at an applied stress of 520 MPa broke after only 2,689,952 cycles. SEM-images of sample C25 shows an inclusion at the fracture surface. In EDX analysis, high peaks for Aluminium, Magnesium, Chromium and Oxygen were found. This mixed-oxide inclusion was identified as the crack initiation point for the early failure of this sample. An SEM-image of sample D35 also shows an inclusion at the fracture surface. Again, in EDX analysis, high peaks for Aluminium, Magnesium, Chromium and Oxygen were found. Also this mixed-oxide inclusion can be identified as the crack initiation point for the early failure of this sample. SEM investigations of samples C31 and D27 also showed oxide-inclusions at the fracture surface which were identified causing the early failure. For both samples, the same elements (Aluminium, Magnesium, Chromium, Oxygen) show high peaks in EDX analysis for these two samples.

**Table 12**

| stress level [MPa] | Example 1 (inventive) (Lot A) | Example 1 (inventive) (Lot B) | Example 2 (comparative) (Lot C) | Example 2 (comparative) (Lot D) |
|---|---|---|---|---|
| | No. of cycles | No. of cycles | No. of cycles | No. of cycles |
| 1430 | 21092 | 66720 | 20700 | 35130 |
| 1430 | 12740 | 63781 | 22140 | 57120 |
| 1430 | 28919 | 59140 | 36120 | 32460 |
| 1430 | 19334 | 37942 | 23550 | 35010 |
| 1430 | 18119 | 39178 | 18270 | 28996 |
| 1430 | 21983 | 22380 | 18150 | 33313 |
| 1040 | 38670 | 128644 | 136680 | 149971 |
| 1040 | 58474 | 218106 | 1004289 | 308580 |
| 1040 | 46980 | 194108 | 8656363 | 80766 |
| 1040 | 34806 | 41310 | 107640 | 178770 |
| 1040 | 38045 | 539089 | 7856526 | 74323 |
| 1040 | 39120 | 290101 | 6852041 | 127650 |
| 880 | 5888420 | 4715690 | 39485962 | 72523366 |
| 880 | 4233055 | 5816670 | 102020453 | 100000000 |
| 880 | 6324748 | 2144125 | 100800000 | 6171230 |
| 880 | 13571905 | 2068519 | 114000000 | 4407264 |
| 880 | 8824316 | 1725656 | 101000000 | 102000000 |
| 880 | 7680042 | 1815390 | 100000000 | 57462763 |
| 800 | 104700000 | 40051186 | - | - |
| 800 | 96874223 | 16938278 | - | - |
| 800 | 59716187 | 26518613 | - | - |
| 800 | 54411683 | 79084889 | - | - |
| 800 | 64971417 | 46467823 | - | - |
| 800 | 100000000 | 24231864 | - | - |
| 700 | 103000000 | 105000000 | 71790 | 100000000 |
| 700 | 109852488 | 97119288 | 47547540 | 100000000 |
| 700 | 101589761 | 107000000 | 100000000 | 549227 |
| 700 | 101623566 | 104000000 | 100000000 | 101337563 |
| 700 | 102000000 | 101000000 | 41282990 | 100000000 |

(continued)

| stress level [MPa] | Example 1 (inventive) (Lot A) | Example 1 (inventive) (Lot B) | Example 2 (comparative) (Lot C) | Example 2 (comparative) (Lot D) |
|---|---|---|---|---|
| | No. of cycles | No. of cycles | No. of cycles | No. of cycles |
| 700 | 103000000 | 103000000 | 100000000 | 100000000 |
| 520 | 100000000 | 100000000 | 145260 | 100000000 |
| 520 | 118987000 | 106000000 | 110800000 | 110800000 |
| 520 | 102064330 | 102000000 | 101000000 | 101000000 |
| 520 | 100963860 | 100613526 | 100500000 | 100500000 |
| 520 | 100845911 | 100845911 | 108000000 | 2689952 |
| 520 | 101009712 | 101006089 | 100000000 | 112000000 |

These fatigue test results are plotted in Figure 12. As can be seen from this plot, lots C and D (comparative) show significantly more undesired outliers that for lots A and B (inventive).

Three lots of example 1a/Ag28% wire (diameter 127 $\mu$m) were also tested against three lots of example 2a wire (same diameter - 127 $\mu$m). All six wire lots have comparable mechanical properties (UTS of 1456 - 1475 MPa).

**Table 13**

| - | Example 1a + 28 wt. % Ag (inventive) | | | Example 2a + 28 wt. % Ag (comparative) | | |
|---|---|---|---|---|---|---|
| Batch | Lot E | Lot F | Lot G | Lot H | Lot J (Fig. 10 & 11) | Lot K |
| UTS [MPa] | 1456 | 1469 | 1474 | 1460 | 1462 | 1475 |
| YM [GPa] | 121 | 121 | 122 | 121 | 122 | 122 |
| Elongation [%] | 2.2 | 2.3 | 2.3 | 2.1 | 2.0 | 2.3 |

At an applied stress of 414 MPa, the wire of all four lots reached the fatigue endurance limit, means the wire does not fail and tests are stopped after 100 Million cycles. While example 1a /Ag28% wire showed no outliers at 414 MPa and below, 4 samples of example 2a /Ag28% wire failed at less than 1.4 Million cycles. All other samples tested at an applied stress of 414 MPa and below survived 100 Million cycles without rupture. For example 2a /Ag28% wire lot H, sample H24 tested at an applied stress of 414 MPa broke after only 1,041,679 cycles. Sample J18 tested at an applied stress of 518 MPa broke after 588,028 cycles and sample J23 tested at an applied stress of 414 MPa broke after 263,488 cycles. Sample K24 tested at an applied stress of 414 MPa broke after 1,355,189 cycles. As an example, SEM-images of sample J23 show an inclusion at the fracture surface. In EDX analysis, high peaks for Aluminium, Magnesium, Chromium and Oxygen were found. This mixed-oxide inclusion was identified as the crack initiation point for the early failure of this sample. SEM investigations of samples H24, J18 and K24 also showed oxide-inclusions at the fracture surface which were identified causing the early failure. For all three samples, the same elements (Aluminium, Magnesium, Chromium, Oxygen) show high peaks in EDX analysis for these three samples.

**Table 14**

| | Example 1a + 28 wt. % Ag | | | Example 2a + 28 wt. % Ag | | |
|---|---|---|---|---|---|---|
| | lot E | lot F | lot G | lot H | lot J | lot K |
| stress level [MPa] | No. of cycles | No. of cycles | No. of cycles | No. of cycles | No. of cycles | No. of cycles |
| 969 | 31,887 | 33,065 | 21,487 | 47,982 | 38,148 | 32,926 |
| 969 | 29,321 | 28,116 | 23,461 | 43,661 | 41,085 | 20,818 |
| 969 | 32,555 | 29,941 | 28,763 | 30,298 | 33,187 | 32,299 |
| 969 | 26,918 | 23,418 | 18,464 | 37,888 | 36,247 | 38,901 |
| 969 | 22,089 | 30,467 | 20,198 | 43,092 | 41,944 | 42,978 |

(continued)

| | Example 1a + 28 wt. % Ag | | | Example 2a + 28 wt. % Ag | | |
| | lot E | lot F | lot G | lot H | lot J | lot K |
| stress level [MPa] | No. of cycles | No. of cycles | No. of cycles | No. of cycles | No. of cycles | No. of cycles |
|---|---|---|---|---|---|---|
| 725 | 246,766 | 231,412 | 114,746 | 74,414 | 235,494 | 109,597 |
| 725 | 199,054 | 189,441 | 123,746 | 79,498 | 128,377 | 92,419 |
| 725 | 287,665 | 262,994 | 168,374 | 94,638 | 118,922 | 91,877 |
| 725 | 200,822 | 186,242 | 145,355 | 75,062 | 162,522 | 102,834 |
| 725 | 500,045 | 290,377 | 169,176 | 62,082 | 238,611 | 99,864 |
| 580 | 1,405,296 | 1,612,743 | 979,651 | 409,644 | 6,780,968 | 311,974 |
| 580 | 1,077,510 | 1,131,168 | 1,846,396 | 668,132 | 12,545,505 | 8,369,715 |
| 580 | 8,201,513 | 993,416 | 1,684,673 | 1,031,447 | 1,945,002 | 3,001,478 |
| 580 | 2,511,763 | 2,197,173 | 639,464 | 342,282 | 2,639,912 | 219,634 |
| 580 | 841,436 | 884,196 | 2,076,465 | 3,539,353 | 8,566,249 | 2,009,899 |
| 518 | 40,051,186 | 86,414,732 | 71,763,385 | 100,000,000 | 100,000,000 | 100,000,000 |
| 518 | 100,000,000 | 78,411,674 | 83,944,821 | 100,000,000 | 100,000,000 | 100,000,000 |
| 518 | 79,084,889 | 100,000,000 | 35,946,337 | 100,000,000 | 588,028 | 100,000,000 |
| 518 | 46,467,823 | 100,000,000 | 100,000,000 | 100,000,000 | 100,000,000 | 100,000,000 |
| 518 | 100,000,000 | 87,867,423 | 54,676,179 | 100,000,000 | 100,000,000 | 100,000,000 |
| 414 | 100,000,000 | 100,000,000 | 100,000,000 | 100,000,000 | 100,000,000 | 100,000,000 |
| 414 | 100,000,000 | 100,000,000 | 100,000,000 | 100,000,000 | 100,000,000 | 100,000,000 |
| 414 | 100,000,000 | 100,000,000 | 98,674,345 | 100,000,000 | 263,488 | 100,000,000 |
| 414 | 100,000,000 | 100,000,000 | 100,000,000 | 1,041,679 | 100,000,000 | 1,355,189 |
| 414 | 100,000,000 | 100,000,000 | 96,674,523 | 100,000,000 | 100,000,000 | 100,000,000 |
| 329 | 100,000,000 | 100,000,000 | 100,000,000 | - | - | - |
| 329 | 100,000,000 | 100,000,000 | 100,000,000 | - | - | - |
| 329 | 100,000,000 | 100,000,000 | 100,000,000 | - | - | - |
| 329 | 100,000,000 | 100,000,000 | 100,000,000 | - | - | - |
| 329 | 100,000,000 | 100,000,000 | 100,000,000 | - | - | - |

These fatigue test results are plotted in Figure 13. As can be seen from this plot, lots H, J and K (comparative) show significantly more undesired outliers that for lots E, F and G (inventive).

Pacemaker lead

A wire with thickness 25 $\mu$m was prepared according to the method described above and with compositions of the alloy as given in table 3. The wires were arranged into a lead as described in figure 1. The leads were tested for fatigue resistance and for impurity inclusions. The results are shown in table 15.

**Table 15**

| Example | Fatigue resistance | Purity from inclusions |
|---|---|---|
| Heat 1 | ++ | ++ |
| Heat 2 | ++ | ++ |

(continued)

| Example | Fatigue resistance | Purity from inclusions |
|---|---|---|
| Heat 3 | ++ | ++ |
| Commercial heat | - | - |
| ++ = very good, - = poor | | |

Reference list

| | |
|---|---|
| 1 | passivated product |
| 2 | raw product |
| 3 | passivation coating |
| 10 | Wire |
| 20 | First region |
| 30 | Further region |
| 50 | Pacemaker |
| 60 | Electrode unit |
| 70 | Electronic unit |
| 100 | Cable |
| 110 | First mass |
| 120 | Body |
| 130 | Precursor |
| 140 | Lead/Cable bundle |
| C | chuck to bushing distance |
| H | height of loop |
| R | minimum of radius of curvature |
| P | bushing load or lateral force at the chuck |
| #A1 | Dark inclusion |

**Claims**

1. A method for manufacturing a passivated product (1), comprising the steps of:

   - providing a raw product (2), and
   - passivating a surface of the raw product (2) at least partially to provide a passivated product (1) with a passivation coating (3),

   wherein the raw product (2) is made at least partially of an alloy comprising the following alloy components:

   a) Cr in the range from about 10 to about 30 wt. %;
   b) Ni in the range from about 20 to about 50 wt. %;
   c) Mo in the range from about 2 to about 20 wt. %;
   d) Co in the range from about 10 to about 50 wt. %;

   wherein the Al content of the alloy is less than about 0.01 wt. %; and
   wherein each wt. % is based on the total weight of the alloy.

2. Method according to claim 1, wherein the Cr, Ni, Mo and Co components are major constituents of the alloy with at least about 95 wt. % of the alloy being Cr, Ni, Mo and Co.

3. Method according to one of the preceding claims, wherein the product is a wire and the method further comprises a wire drawing of the passivated wire.

4. Method according to the preceding claim, wherein the passivation coating (3) is configured to provide a full coverage of the surface of the raw wire, which is maintained during wire drawing and prevents a direct contact between the raw wire and a drawing tool.

5. Method according to claim 3 or 4, further comprising an additional passivating of the drawn wire and an additional wire drawing of the again passivated wire.

6. Method according to one of the preceding claims, wherein the passivation coating (3) has a thickness in the range of 10 nm to 50 $\mu$m, and preferably in the range of 50 nm to 10 $\mu$m.

7. Method according to one of the preceding claims, wherein the passivating is done by means of a nitric acid solution.

8. Method according to one of the preceding claims, wherein the passivation coating (3) is inert.

9. Method according to one of the preceding claims, wherein the passivation coating (3) is provided directly on the metallic surface of the raw product (2).

10. Method according to one of the preceding claims, wherein the passivation coating (3) is an oxide layer of an oxide of one of the group of Cr, Ni, Mo and Co.

11. Method according to the preceding claim, wherein the passivation coating (3) is a chromium oxide layer.

12. Method according to one of the claims 1 to 9, wherein the passivation coating (3) is an oxide layer of an oxide of a noble metal.

13. Method according to the preceding claim, wherein the passivation coating (3) is a platinum oxide layer.

14. Method according to one of the preceding claims, further comprising a cleaning of the raw product (2) before passivating.

15. Method according to the preceding claim, wherein the cleaning comprises one of a group of organic solvent degassing, hot alkaline cleaning and acidic cleaning.

16. Method according to one of the preceding claims, further comprising a neutralizing of the raw product (2) after cleaning and before passivating.

17. Method according to one of the preceding claims, further comprising a rinsing of the passivated product (1) after passivating and an electrical monitoring of the rinsing agent after rinsing.

18. Method according to one of the preceding claims, wherein the raw product (2) is annealed before passivating.

19. Method according to one of the preceding claims, further comprising a removing of the passivation coating (3), a heat treating of the product and a re-passivating of the heat-treated product.

20. A passivated product (1), comprising a raw product (2) and a passivation coating (3), wherein the passivation coating (3) is provided at least partially on a surface of the raw product (2),
wherein the raw product (2) is made at least partially of an alloy comprising the following alloy components:

   a) Cr in the range from about 10 to about 30 wt. %;
   b) Ni in the range from about 20 to about 50 wt. %;
   c) Mo in the range from about 2 to about 20 wt. %;
   d) Co in the range from about 10 to about 50 wt. %;

wherein the Al content of the alloy is less than about 0.01 wt. %; and
wherein each wt. % is based on the total weight of the alloy.

21. A medical device comprising a passivated product (1) according to the preceding claim as a lead.

Fig. 1

Fig. 2

Fig. 3

Fig. 4

Fig. 5

Fig. 6

Fig. 7

Fig. 8

EP 3 415 651 A1

Fig. 9

Fig. 10

31

EP 3 415 651 A1

Fig. 11

Fig. 12

Fig. 13

Fig. 14

# EUROPEAN SEARCH REPORT

**Application Number**

EP 17 17 5929

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | WO 2005/026399 A1 (ATI PROPERTIES INC [US]; FORT WAYNE METALS RES PROD [US]; FORBES JONES) 24 March 2005 (2005-03-24) <br> * claims 1,48,49 * <br> * examples WE52, WE53, WE54, WE48; table 2 * <br> * examples Heat-WF64, Heat-WF66; table 10 * <br> * page 3, line 19 - page 4, line 8 * <br> * page 2, line 10 - page 3, line 11 * | 1-21 | INV. <br> C22C19/05 |
| Y | US 2015/099958 A1 (SHAN NICOLAS [FR] ET AL) 9 April 2015 (2015-04-09) <br> * paragraph [0002] * <br> * paragraphs [0026], [0066] * <br> * paragraphs [0030], [0078] * <br> * paragraphs [0032], [0082] * <br> * paragraph [0119] * <br> * figures 1a-1d,2,3,10a-10c * | 1-21 | |
| X | WO 2016/064790 A1 (MEDTRONIC INC [US]) 28 April 2016 (2016-04-28) <br> * page 2, paragraphs 1,3 * <br> * page 5, lines 6-8,22 - line 26 * <br> * page 7, line 12 - line 19 * <br> * page 10, paragraphs 2,3 * <br> * page 11, paragraphs 1-3, 18 - paragraph 22 * <br> * page 12, paragraph 5 * <br> * page 12, line 26 - page 13, line 6 * <br> * page 14, paragraph 1 * <br> * figures 1,2,11 * | 1-21 | **TECHNICAL FIELDS SEARCHED (IPC)** <br><br> C22C |

The present search report has been drawn up for all claims

| Place of search | Date of completion of the search | Examiner |
|---|---|---|
| Munich | 12 December 2017 | Rolle, Susett |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

**ANNEX TO THE EUROPEAN SEARCH REPORT
ON EUROPEAN PATENT APPLICATION NO.**

EP 17 17 5929

This annex lists the patent family members relating to the patent documents cited in the above-mentioned European search report.
The members are as contained in the European Patent Office EDP file on
The European Patent Office is in no way liable for these particulars which are merely given for the purpose of information.

12-12-2017

| Patent document cited in search report | | | Publication date | Patent family member(s) | | | Publication date |
|---|---|---|---|---|---|---|---|
| WO 2005026399 | A1 | | 24-03-2005 | AT | 554192 | T | 15-05-2012 |
| | | | | CN | 1867687 | A | 22-11-2006 |
| | | | | DK | 1664360 | T3 | 14-05-2012 |
| | | | | EP | 1664360 | A1 | 07-06-2006 |
| | | | | HK | 1092842 | A1 | 11-01-2013 |
| | | | | JP | 5165241 | B2 | 21-03-2013 |
| | | | | JP | 2007504362 | A | 01-03-2007 |
| | | | | US | 2005051243 | A1 | 10-03-2005 |
| | | | | WO | 2005026399 | A1 | 24-03-2005 |
| US 2015099958 | A1 | | 09-04-2015 | CN | 103143114 | A | 12-06-2013 |
| | | | | EP | 2581107 | A1 | 17-04-2013 |
| | | | | US | 2013096658 | A1 | 18-04-2013 |
| | | | | US | 2015099958 | A1 | 09-04-2015 |
| WO 2016064790 | A1 | | 28-04-2016 | CN | 107077907 | A | 18-08-2017 |
| | | | | EP | 3209344 | A1 | 30-08-2017 |
| | | | | US | 2016111178 | A1 | 21-04-2016 |
| | | | | WO | 2016064790 | A1 | 28-04-2016 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2005026399 A1

- US 20050051243 A1